# EUROPEAN PATENT APPLICATION

(11) **EP 3 988 669 A1**
(43) Date of publication of application: **27.04.2022**
(21) Application number: 20203357.7
(22) Date of filing: 22.10.2020
(51) Int. Cl.: C12Q 1/6813, C12Q 1/6841

(54) **METHOD FOR NUCLEIC ACID DETECTION BY OLIGO HYBRIDIZATION AND PCR-BASED AMPLIFICATION**

(71) Applicant: Max-Delbrück-Centrum für Molekulare Medizin in der Helmholtz-Gemeinschaft, 13125 Berlin (DE)
(72) Inventor: Pombo, Ana, 10715 Berlin (DE); Sparks, Thomas, 13125 Berlin (DE)
(74) Representative: Moré, Solveig Helga

(57) **Abstract**

The present invention relates to the field of nucleic acid sequencing at the single cell level, e.g., single-cell RNA sequencing (scRNA-seq). In particular, the invention provides a method of detecting nucleic acid in a fixated or non-fixated nucleic acid-containing compartment such as a eukaryotic cell or nucleus thereof, by hybridizing a plurality of single-stranded (ss)DNA oligonucleotide probes to complementary nucleic acid molecules within said compartment; removing ssDNA oligonucleotide probes from the compartment that have not specifically hybridized to nucleic acid; and identifying the ssDNA oligonucleotide probes specifically hybridized to nucleic acid molecules within said compartment by sequencing or amplification, thereby determining the corresponding nucleic acids present in said compartment. The method does not require a step of sequential ssDNA probe hybridization to the same target nucleic acid as a means for increased specificity or sensitivity, and preferably further does not require steps of RNA isolation and cDNA generation. The method of the invention has the potential to detect substantially every known and/or unknown nucleic acid species, in particular RNA, e.g., protein-encoding mRNAs as well as non-coding RNAs. The method further enables spatial mapping of detected nucleic acids, wherein the compartment is sectioned or dissociated into a single cell suspension prior to probe hybridization to obtain a collection of fractions and thus nucleic acid molecules are separated from each other depending on their localization or which cell type they belonged to. Spatial mapping of detected nucleic acids may be combined with the detection of at least one DNA locus, at least one protein, or with the analysis of chromatin condensation. The method of the invention is designated oligo-seq.

SEQUENCE LISTING

<110> Max-Delbrueck-Centrum fuer Molekulare Medizin

<120> Method for nucleic acid detection by oligo hybridization and PCR-based
amplification

<130> 12576 P 6581 EP

<160> 6

<170> BiSSAP 1.3.6

<210> 1
<211> 34
<212> DNA
<213> Artificial Sequence

<220>
<223> GAT-7N

<220>
<221> primer_bind
<222> 28..34
<223> /note="WGA degenerated primer sequence"

<400> 1
gtgagtgatg gttgaggtag tgtggagnnn nnnn                                    34

<210> 2
<211> 27
<212> DNA
<213> Artificial Sequence

<220>
<223> GAT-COM

<400> 2
gtgagtgatg gttgaggtag tgtggag                                            27

<210> 3
<211> 20
<212> DNA
<213> Artificial Sequence

<220>
<223> Probe primer Forward

<400> 3
ggcacaacgt tgcagcacag                                                    20

<210> 4
<211> 20
<212> DNA
<213> Artificial Sequence

<220>
<223> Probe primer Reverse

<400> 4
caccaacgct accagctccg                                                    20


<210> 5
<211> 53
<212> DNA
<213> Artificial Sequence


<220>
<223> Probe Primer A MeRev Forward

<400> 5
tcgtcggcag cgtcagatgt gtataagaga cagggcacaa cgttgcagca cag              53


<210> 6
<211> 54
<212> DNA
<213> Artificial Sequence


<220>
<223> Probe Primer B MeRev Reverse

<400> 6
gtctcgtggg ctcggagatg tgtataagag acagcaccaa cgctaccagc tccg             54

## Description

The present invention relates to the field of nucleic acid sequencing at the single cell level, e.g., single-cell RNA sequencing (scRNA-seq). In particular, the invention provides a method of detecting nucleic acid in a fixated or non-fixated nucleic acid-containing compartment such as a eukaryotic cell or nucleus thereof, by hybridizing a plurality of single-stranded (ss)DNA oligonucleotide probes to complementary nucleic acid molecules within said compartment; removing ssDNA oligonucleotide probes from the compartment that have not specifically hybridized to nucleic acid; and identifying the ssDNA oligonucleotide probes specifically hybridized to nucleic acid molecules within said compartment by sequencing or amplification, thereby determining the corresponding nucleic acids present in said compartment. The method does not require a step of sequential ssDNA probe hybridization to the same target nucleic acid as a means for increased specificity or sensitivity, or steps of RNA isolation and cDNA generation. The method of the invention has the potential to detect substantially every known and/or unknown nucleic acid species, in particular RNA, e.g., protein-encoding mRNAs as well as non-coding RNAs. The method further has sufficient sensitivity to detect low abundance nucleic acids and their abundance in subcellular compartments. The method further enables spatial mapping of detected nucleic acids, wherein the compartment is sectioned prior to probe hybridization to obtain a collection of fractions and thus nucleic acid molecules are separated from each other depending on their localization. Spatial mapping of detected nucleic acids may be combined with the detection of at least one DNA locus, at least one protein, or with the analysis of chromatin condensation, chromatin contacts and chromatin radial position in the cell nucleus.

Ribonucleic acid (RNA) is a highly versatile molecule with numerous biological functions. Best known for transmitting genetic information from DNA to the sites of protein biosynthesis, the ribosomes, RNA molecules may also exhibit regulatory, catalytic or processing activities. Understanding when, where, how and why RNA molecules are expressed in cells is thus of major interest to researchers and clinicians alike.

In consequence, numerous methods for detecting, identifying and quantifying RNAs in individual cells, tissues or whole organisms have been developed and continuously optimized.

Northern blotting is a method wherein RNAs are first separated by size via electrophoresis before being transferred to a membrane. RNA detection is then achieved by adding labeled probes to the membrane that hybridize to RNAs of interest (Josefsen et al., 2011, Northern Blotting Analysis. Methods Mol Biol. 703, 87-105).

Nuclease protection assays rely on radiolabeled or non-isotopic probes that anneal to RNAs in a sample. After hybridization, single-stranded unhybridized probes and RNAs are degraded by nucleases, while probe-bound RNA fragments are retained to become subsequently separated and detected on an acrylamide gel via autoradiography (https://www.thermofisher.com/de/de/home/references/ambion-tech-support/ribonuclease-protection-assays/general-articles/the-basics-what-is-a-nuclease-protection-assay.html).

The discovery of reverse transcriptase, a retrovirus-derived enzyme that enables reverse transcription of RNA molecules into complementary DNA (cDNA), was a major breakthrough for RNA research. The conversion of RNA into more stable cDNA reduces the rate of sample degradation, and facilitates handling of the biological material, thus allowing for the development and application of more sophisticated RNA detection methods. Reverse transcription quantitative polymerase chain reaction (RT-qPCR) for instance is a PCR-based method wherein RNA is first reverse transcribed into cDNA and subsequently amplified using specifically designed primer pairs that anneal to cDNA molecules of interest. It requires sufficient RNA integrity to achieve polymerization of the cDNA to lengths compatible with further amplification (about >100 n, or typically >200nts). Quantification of cDNA may involve the use of PCR amplification in the presence of e.g. fluorescent dyes such as SYBR Green that intercalates with the cDNA and is detected by a sensor inside the qPCR thermocycler. After each PCR cycle, the increase of cDNA copy number leads to increasing fluorescent signal until a certain threshold is reached. After completion of a predetermined number of PCR cycles, a computer then uses this information to calculate the amount of starting RNA for each gene tested in a sample.

None of the above-mentioned methods however allows for global transcriptome-wide analyses of cells. A massive, high-throughput parallel detection of thousands of RNAs extracted from biological samples was for the first time made possible with the development of hybridization-based DNA microarrays. Briefly, fluorescently labeled cDNA fragments that have been obtained by reverse transcription of isolated RNA are hybridized via complementary base pairing to single-stranded DNA oligonucleotide probes fixed to defined regions (features) on the surface of a microchip. Each of these features contains probes for a particular gene of interest. Bound cDNA sequences generate a fluorescent signal. The strength of this signal depends on the amount of target cDNA bound to the probes in a given feature of the chip. The intensity detected for a respective feature is compared to that of the corresponding feature under a different condition, e.g., when the biological sample has been treated with an environmental stimulus. In consequence, changes in expression of the tested genes can be relatively quantified (Miller et al., 2009, Basic Concepts of Microarrays and Potential Applications in Clinical Microbiology., Clin Microbiol Rev. 22(4): 611-633).

The improvement and increasing affordability of next generation sequencing (NGS) technologies led to a gradual replacement of microarrays by RNA sequencing (RNA-seq). Despite its name, RNA-seq usually does not involve direct sequencing of RNA molecules. In fact, conventional RNA-seq methods sequence cDNA fragments of approximately 200 nt derived from RNA isolated from biological materials. cDNA fragments are ligated to specific sequencing adaptors to obtain a sequencing library. The library is next PCR-amplified prior to a fragment size selection step. In some variations of RNA-Seq, highly abundant structural RNAs such as ribosomal RNAs (which can compromise 90% of the total RNA), are positively selected against, adding an additional costly step to the procedure. Different working platforms are known in the art for conducting the actual sequencing. The most commonly used sequencing platforms include Illumina, Roche 454, Helicos, PacBio, SOLiD and Complete Genomics. Illumina, Roche 454 or PacBio are based on sequencing-by-synthesis and utilize fluorescently-labeled deoxynucleoside triphosphates (dNTP) that can be detected by the sequencing device. Briefly, one of four different fluorescently-labeled dNTPs is added to a growing nucleic acid chain that is complementary to the nucleotide sequence of the cDNA fragment to be a sequenced. The fluorescent label of each dNTP terminates further polymerization of the chain until the fluorescent dye has been imaged and enzymatically removed. Afterwards another labeled dNTP can be added to the chain. Based on the fluorescent signal and its intensity, the sequencing device is able to call the correct bases corresponding to the template cDNA (https://www.illumina.com/documents/products/techspotlights/techspotlight_sequencing.pdf). Such kind of short-read cDNA sequencing approaches sequence a library to an average of 20-30 million reads per sample (Stark et al., 2019, RNA sequencing: the teenage years. Nature Reviews Genetics 20, 631-656). Alternative approaches used by other sequencing platforms differ from the classical sequencing-by-synthesis method and may employ e.g. sequencing-by-ligation (e.g. SOLiD) or Nanopore Sequencing technologies.

For subsequent bioinformatic analysis of the raw sequencing data, sequencing reads are tested first for their quality before being mapped to a reference genome to assemble the transcriptome. If a reference genome is not available, de-novo assembly of sequencing reads may be performed.

Recent years have witnessed a rapid improvement in the field of RNA-seq. New methods that allow for sequencing of long non-fragmented cDNA were developed as well as direct RNA sequencing which omits the intermediate step of reverse transcription (Stark et al., 2019). The transcriptome-wide analysis of individual cells by single cell RNA (scRNA) sequencing posed a major challenge in recent years. ScRNA-seq enables researchers to study e.g. rare cell types whose unique transcriptional profiles are often obscured by other, more abundant cell types in bulk samples. ScRNA-seq has facilitated the identification of previously unknown cells on the basis of their unique transcriptional signatures. Different techniques and methods suitable for scRNA-seq are known to vary in sensitivity, precision, number of cells to be analyzed and monetary costs, and thus may be used for different applications and experimental setups (Ziegenhain et al., 2017, Comparative Analysis of Single-Cell RNA Sequencing Methods. Mol Cell. 85(4), 631-643). In general, individual cells are isolated in single wells or microfluidic droplets. Each well or droplet contains all required chemicals to lyse the cell and prepare a sequencing library, including the steps of RNA isolation, reverse transcription and adaptor ligation (Ziegenhain et al., 2017).

Transcriptome analyses in single cells face several important challenges: First of all, deep-sequencing of RNA requires a minimum amount of starting material. The amounts of RNA that can be isolated from single cells is however limited and RNA is highly prone to degradation during isolation. As a consequence, the informative value of conventional scRNA-seq methods is limited by the availability of the starting material. Reverse transcription of the isolated scRNAs, along with prior amplification steps, such as "linear amplification" transforms the transcriptome information content to more stable cDNA molecules, but often introduces other biases that can ultimately falsify the evaluation of the sequencing results.

Transcriptome studies with limited RNA concentrations collected from individual cells therefore often necessitate the use of alternative RNA detection methods. The use of fluorescently labeled oligonucleotide probes that can be microscopically detected *in situ,* i.e. in their natural cellular environment, thus circumventing the need for RNA isolation, represents a common approach. These fluorescent *in situ* hybridization (FISH) probes localize and hybridize to complementary RNA molecules in a fixed tissue section or a cell. Bound probes can subsequently be detected at subcellular resolution using a fluorescent microscope or a specialized sensory device. Nowadays, extensive libraries of RNA-FISH probes such as Oligopaints (Beliveau et al., 2012, Versatile design and synthesis platform for visualizing genomes with Oligopaint FISH probes. Proc Natl Acad Sci 109(52), 21301-6) may be bioinformatically designed to cover most of the genome, and they can be generated by massive parallel synthesis. The numbers of genes to be analyzed simultaneously by FISH-based RNA detection methods were however traditionally restricted by the limited amount of fluorescent tags that could be monitored in parallel. This problem was addressed by methods such as e.g. RNASeqFISH+ that are based on modified probes with complex multicolored barcodes that greatly expand the number of genes that can be simultaneously detected in a single experiment.

Similarly, multiplex nCounter assays provided by NanoString Technologies are based on RNA detection via a probe pair consisting of a target-specific capture probe that anneals to an RNA molecule of interest and a gene-specific color-coded reporter probe that hybridizes to the target probe. The RNA-capture-probe-reporter probe complexes are immobilized and aligned on an imaging surface of a specific cartridge. The cartridge is subsequently scanned by a specific microscopic device capable of automated fluorescence detection that directly counts the labeled probes (Geiss et al., 2008, Direct multiplexed measurement of gene expression with color-coded probe pairs. Nat Biotechnol. 26(3), 317-325). However, methods like nCounter require the purchase of special laboratory equipment capable of detecting the probes. The necessary probes furthermore bind only to selected panels of mRNAs and miRNAs and cannot be simply generated in the lab.

Based on their nCounter assay, NanoString Technologies further developed a method known as GeoMx^{™} Digital Spatial Profiling (DSP). The method involves co-staining a tissue or cell section with fluorescent markers and oligonucleotide "profiling" probes against RNAs. Each probe is formed by a target complementary sequence that is attached to a DSP oligonucleotide barcode via a photocleavable linker. Individually chosen regions of interest of the tissue/cell section are next illuminated with UV-light to release the DSP-oligo-sequences from the section. The DSP-oligonucleotides are subsequently aspirated and transferred into wells of a microtiter plate. The information within each well is indexed to each of the previously selected regions of interest on the tissue. Finally, the DSP-oligonucleotides are hybridized to NanoString barcodes and are quantitated on the nCounter platform (GeoMx^{™} product brochure, available on https://www.nanostring.com/products/geomx-digital-spatial-profiler/geomx-dsp). Therefore, the GeoMx^{™} technology, too, requires the purchase of expensive equipment as well as the use of specifically developed software suites, and specialized probes containing DSP.

WO2019/157445 and Merritt et al., 2020 (Multiplex digital spatial profiling of proteins and RNA in fixed tissue. Nature Biotechnology 38, 586-599) relate to the above described method of DSP in more detail and also mention probe identification and quantification via NGS as an alternative to the nCounter platform. For NGS analysis, the DSP oligonucleotides (referred to as "identifier nucleotides" in WO2019/157445 or as "indexing oligonucleotides" in Merritt et al.) may comprise two primer binding sites for their amplification and the addition of sequencing adaptors as well as a unique molecular identifier, and a specific nucleic acid sequence which identifies the RNA target (i.e. a barcode sequence). After their UV-induced release from the target complementary sequence, the DSP oligonucleotides are subsequently PCR amplified and sequenced. Therefore, the method circumvents the need for reverse transcription of RNA and relies on NGS for probe identification. However, the method is technically very challenging, as it requires a rather complex probe design. The unique barcode sequences present in the DSP oligonucleotides further have the potential to cause off target annealing events, thus reducing the specificity of the assay. This problem is further exacerbated by the fact that the barcode sequences are located adjacent to unique molecular identifiers and to primer binding sites. Barcode sequences are also known to introduce an increased risk of PCR bias. In addition, for release of the identifier nucleotide, the sample needs to be exposed to, e.g., UV-light, with potentially harmful consequences to the nucleic acids present in said sample.

Spatial transcriptomic methods such as SlideSeq and/or Visium Spatial Gene Expression (Rodriques et al., 2019. Science 363(6434):1463-1467) combine the use of short oligonucleotide probes and NGS to spatially capture and sequence RNA molecules released from proximal tissue areas. These methods involve the attachment of spatially organized and barcoded oligo d(T) primers or DNA-barcoded microparticles to the surface of microscope slides in an organized/localized/arrayed manner. When a cell or tissue is brought in contact with these slides and treated to release its RNA content by osmosis, the primers capture mRNA molecules that diffuse into their vicinity. Captured mRNAs are reverse transcribed into cDNAs that incorporate the spatial barcode of their primers and are subsequently sequenced. During the following analysis, the barcodes allow to retrace the subcellular region where a detected RNA has originally been found (Stahl et al., 2016, Visualization and analysis of gene expression in tissue sections by spatial transcriptomics. Science 353(6294), 78-82).

In a recently published pre-print, Marshall et al. introduced a method termed Hybridization of Probes to RNA for sequencing (HyPR-seq; Marshal et al., 2020, HyPR-seq: Single-cell quantification of chosen RNAs via hybridization and sequencing of DNA probes, BioRxiv preprint doi: https://doi.org/10.1101/2020.06.01.128314). The method is based on the hybridization chain reaction (HCR) smFISH protocol. In brief, HyPR-seq first requires two ssDNA initiator probes to anneal to target RNAs via homologous base-pairing. In a second step, said initiator probes serve as binding sites for a short hairpin oligo probe to which, subsequently, a special "read-out" probe is hybridized. Said "read-out" probe is next ligated to the 5' end of the initiator probe to ultimately obtain a single ssDNA fragment, which is first amplified by PCR and subsequently sequenced. Therefore, while HyPR-seq is an NGS-based technique that circumvents the need for RNA isolation and reverse transcription, transcript detection relies on a three-layered, sequential hybridization of at least four distinct oligonucleotides, thus limiting the number of genes that can be analyzed simultaneously in a single experiment to not more than 100 (Marshal et al., 2020). At present, no method for RNA detection is available that relies on next generation sequencing but does not require either the steps of RNA isolation, cDNA generation or signal amplification by consecutive hybridizations of complementary oligonucleotide probes. With conventional RNA-seq methods, RNA degradation and reverse transcription biases cannot be avoided and must be taken into account when assessing the raw sequencing data during bioinformatic evaluation. Accordingly, there is a risk of introducing biases into the analysis, especially when the data is derived from low quantities of starting material such as scRNA, due to stochastic sampling of the RNA content of each cell. In contrast, many FISH-based methods relying on the use of oligonucleotide probes for detection of RNA transcripts may detect RNAs even at subcellular resolution, but they are either limited by the number of genes or transcripts that can be monitored simultaneously, or by the high expenses associated with the purchase of specialized equipment and reagents.

In light of the state of the art, the present inventors addressed the problem of providing a novel and highly sensitive method for RNA detection at single-cell resolution overcoming many of the limitations of presently used methods, and that, advantageously, has the potential to detect and quantify any RNA species at every possible maturation state as well as their alternative splicing variants, isoforms, fusion products or single nucleotide polymorphisms in a sequence-specific manner.

The problem is solved by the present invention, in particular by the subject matter of the claims. The method of the invention is designated Oligo-based Mapping by Sequencing (oligo-seq) or Transcript Oligo-based Mapping by PCR (TOM-PCR). It is suitable not only for detecting RNA, but any type of nucleic acid present in a sample.

The present invention provides a method of detecting a nucleic acid comprising steps of
(a) providing a nucleic acid-containing compartment;
(b) hybridizing at least one single-stranded DNA oligonucleotide probe, preferably, a plurality of single-stranded DNA oligonucleotide probes, to nucleic acid molecules within said compartment;
(c) removing single-stranded DNA oligonucleotide probes from the compartment that have not specifically hybridized to any nucleic acid within the compartment;
(d) identifying the single-stranded DNA-oligonucleotide probes specifically hybridized to nucleic acid molecules within said compartment by probe sequencing or probe amplification; and thus, determining nucleic acids corresponding to the probe present in said compartment,
   wherein the method does not comprise sequential probe hybridization as a means to amplify nucleic acid detection.

Nucleic acids are biopolymers formed of monomeric building blocks called nucleotides. Each nucleotide consists of a 5-carbon sugar, a phosphate group and a nitrogenous base. In the present invention, the term nucleic acid refers to naturally occurring nucleic acids, i.e., either deoxyribonucleic acid (DNA) or ribonucleic acid (RNA). The method of the invention can, with particular advantages, be used for detecting RNA. Thus, preferably, throughout the invention, the nucleic acid is RNA.

DNA mostly exists in a double-stranded state and takes a characteristic double-helical shape in cells. In rare cases, DNA may however also exist in a single-stranded state, e.g., as part of an intermediate structure formed during DNA transcription called R-loop and during DNA replication. In addition, ssDNA viruses encode their genetic information in a single-stranded circular DNA molecule.

Ribonucleic acid (RNA) is a polymeric molecule that is assembled as a chain of nucleotides formed by the sugar ribose, a phosphate group and one of the four nucleobases adenine (A), guanine (G), cytosine (C) and uracil (U). In contrast to most naturally occurring DNAs, RNAs are single-stranded molecules that can form complex secondary and tertiary structures via intramolecular base pairings. Depending on their function, RNAs can be assigned to different sub-classes and species.

Detecting a nucleic acid is herein understood to mean that the method of the invention enables the skilled person to understand whether a given nucleic acid is present in a sample or not. Detecting may also encompass quantifying a nucleic acid, i.e. finding how many copies of a given nucleic acid are present in a sample. It may further comprise the comparison of nucleic acid quantities, i.e., testing whether a nucleic acid is more abundant than another in one sample, or more abundant compared to another sample. The method of the invention exhibits a sufficiently high sensitivity to detect even nucleic acids rarely occurring in a sample. The nucleic acid to be detected may be e.g. any DNA or RNA of interest. In a preferred embodiment, the nucleic acid to be detected has been endogenously generated within the compartment. The nucleic acid may however also be an exogenous nucleic acid, i.e. it may have been introduced into the compartment from the outside by e.g. transfection or microinjection. The exogenous nucleic acid may be e.g. an artificial short-hairpin RNA (shRNA) designed to knock-down a protein-coding gene. The exogenous nucleic acid to be detected may also naturally be introduced into the compartment, e.g. during viral infection.

The nucleic acid-containing compartment according to the invention may be a tissue within an organ, a eukaryotic cell or cluster of cells, a nucleus of a eukaryotic cell, a nucleolus of a eukaryotic cell, cytoplasm of a eukaryotic cell, a mitochondrion, a chloroplast, an exosome, a prokaryotic cell, or a virus.

The eukaryotic cell may be e.g. a plant cell, a fungal cell or an animal cell. In a preferred embodiment, the eukaryotic cell is a mammalian cell. The mammalian cell may preferably be derived from a human being, such as a cell from a human patient having a disease or disorder, or being diagnosed for a disorder, or a healthy subject. The cell may be, e.g., a tumor cell or a stem cell. Such cells can be especially suitable for use in the present invention, as they exhibit unique and highly characteristic transcriptional signatures that can be readily identified with the herein described method. The mammalian cell may however also be a non-human cell, such as a cell from a mammalian genetic model organism, e.g., a mouse, rat, rabbit, guinea pig, pig or non-human primate.

While the cell preferably is a mammalian cell, such as a human cell, it may also be of interest to investigate, and, optionally, compare, e.g., RNA expression of other organisms, such as *E. coli,* yeast, *A. thaliana, C. elegans, X. laevis, D. rerio, N. furzeri, D. melanogaster* or planarians.

The nucleic acid-containing compartment may also be a prokaryotic cell, i.e. from a bacterium or an archaeon. The invention can also be applied to, e.g., study the spatial organization of microbiota in a biofilm or the intestinal lumen, where FISH has been successfully applied (reviewed in Tropini et al., 2017, The Gut Microbiome: Connecting Spatial Organization to Function. Cell Host & Microbe 21(4), 433-442; Liu et al., 2017, Low-abundant species facilitates specific spatial organization that promotes multispecies biofilm formation. Environ Microbiol. 19, 2893-905; Liu et al., 2019, Deciphering links between bacterial interactions and spatial organization in multispecies biofilms. The ISME Journal 13, 3054-3066).

In an alternative embodiment, the nucleic acid-containing compartment may e.g. be a subcellular structure or organelle. It may for instance be a nucleus of a eukaryotic cell, e.g., a mammalian, preferably, human, cell. The nucleic acid-containing compartment may also be a substructure of a eukaryotic cell nucleus, i.e., a nucleolus. The compartment may also comprise the cytoplasm of a mammalian, e.g., a human cell. While the compartment may comprise only said organelles of the cell, it may alternatively comprise combinations thereof, e.g., it may also be a complete human cell, comprising nucleus, cytoplasm and mitochondria. It may also comprise, e.g., cytoplasm and mitochondria, but not the nucleus. Exosomes are endosome-derived, small membrane-bound extracellular vesicles that are involved in intercellular communication and frequently contain mRNAs and microRNAs (miRNAs) as their cargo.

Cells can be derived from cell culture, or analyzed ex *vivo* from a specific tissue from a living organism or a dead organism, i.e., post-mortem, or from a whole experimental organism (e.g. a whole D. *melanogaster* embryo or any developmental stage of C. *elegans).* The cells may for instance be obtained from sections of brain areas commonly associated with disease. Accordingly, cells may stem from complex tissues that encompass a plurality of different cell types. It is possible that the precise identity of the cell may not even be known by the time of analysis, but can be determined by the method of the invention. The method may even be suitable to identify and describe previously uncharacterized cell types on the basis of unique transcriptional signatures.

The cell to be subjected to the method of the invention may be at any state of the cell cycle. Depending on the experimental aim, it may be helpful to isolate cells at a specific stage of the cell cycle, since transcriptional profiles may vary considerably throughout the cell cycle. When comparing gene expression in a plurality of cells, all cells should preferably be at a common cell cycle stage, e.g., synchronized. The stage may be interphase, e.g., G₁, S or G₂ phase, the mitotic phase, or the cytokinesis phase. Preferably, the stage is interphase.

Alternatively, RNA transcription may also be compared in cells at different cell cycle stages. Cells may also vary with regard to their differentiation state. Cells may e.g. be totipotent or pluripotent stem cells with the potential to differentiate into distinct cell types. Alternatively, cells analyzed by the method of the invention may be fully differentiated and fulfil specific functions in a tissue. The cell may also be any cell in the process of differentiating from a stem cell to a terminally differentiated cell.

The nucleic acid-containing compartment may also be a section of a tissue encompassing multiple cells, or parts thereof.

In one embodiment, the nucleic acid-containing compartment is sectioned before hybridization of the ssDNA oligonucleotide probes according to step (b). This provides a plurality of fractions or sections. Sectioning of the nucleic acid-containing compartment may be achieved by any suitable method known from the state of the art, e.g., ultracryosectioning or cryomilling, preferably ultracryosectioning. The cryosections are preferably produced in the absence of resin-embedding, e.g., by the Tokuyasu method (Tokuyasu, K. T., 1973, A technique for ultracryotomy of cell suspensions and tissues. J. Cell Biol. 57, 551-65). Said method involves cryoprotection of fixed tissues using embedding in a saturated sucrose solution for at least about 30 min, or at least about 2h, or at least about 1 day, or up to 1 week, at a temperature of 0-25°C, preferably, at room temperature (20-25°C) or at about 4°C, e.g., for 2h at room temperature, or for 2h at room temperature followed for short term storage for 1 day up to a week at about 4°C. Embedding is followed by placing the sucrose-embedded cell pellet or tissue, or organism, e.g. on a metal stub which acts as a sample holder, before freezing in liquid nitrogen, and sectioning preferably at -80 to -110°C, depending on cell type or tissue, e.g., about -100°C. Slightly modified methods (Guillot P.V., Xie S.Q., Hollinshead M., Pombo A., 2004 Fixation-induced redistribution of hyperphosphorylated RNA polymerase II in the nucleus of human cells. Exp. Cell Res. 295, 460-468; Pombo A, Hollinshead M, Cook PR, 1999, Bridging the resolution gap: Imaging the same transcription factories in cryosections by light and electron microscopy. J. Histochem. Cytochem. 47, 471-480) have been shown to provide good results. These methods preserve overall cellular architecture comparable to that observed in unfixed cryosections (McDowall et al., 1989, The structure of organelles of the endocytic pathway in hydrated cryosections of cultured cells, Eur. J. Cell Biol. 49, 281 - 294), and provide optimal preservation of active RNA polymerases and nuclear architecture (Guillot et al., 2004). The method of Chen et al., 2014, Small 10:3267, can alternatively be used. Unfixed sections subjected to vitrification may, e.g., be prepared according to methods described by Lučič V., et al., 2013. J. Cell Biol. 202 (3), 407.

Sections, e.g., of a nucleus, can have a thickness of about 70 nm to about 1000 nm, preferably, 150-220 nm or 180-200 nm for a nucleus 5-15 micrometer in diameter. In the context of the invention, a slice thickness of below 300 nm, e.g., 150-220 nm, preferably, about 200 nm is referred to as "ultrathin". Commercial equipment for cryosectioning in a sucrose medium for fixed cells is available (e.g., Leica UltraCut UCT 52 ultracryomicrotome). Sections may alternatively also be 4-10 µm thick cryostat sections (https://www.protocols.io/view/Stellaris-RNA-FISH-Protocol-for-FrozenTissue-iwgs5v), vibratome sections of 50-300 micrometers (https://www.protocols.io/view/exfish-tissue-slice-n6adhae), cells on monolayer, or cells in suspension.

From sections of cells, sections of isolated compartments may be prepared, e.g., nuclear profiles, sectioned cytoplasm in the absence of nuclear components (in particular, to detect RNA), and optionally further in the absence of mitochondrial components, or sections of isolated organelles, such as sections of mitochondria.

Sectioning leads to a collection of fractions, i.e. a plurality of fractions. The optimal thickness of sections depends on the size of the compartment. It may be separated into 5-300 fractions, 10-100 fractions, more preferably, 40-60 fractions or about 45-50 fractions. In some embodiments, the thickness of the fractions may be homogenous for the whole analysis. For other applications, e.g., for quantifying relative amounts of several RNA species, or if there is a means for calibration, e.g., one RNA relative to another (e.g., relative to actin), the thickness of the different slices may also vary within a single compartment. Preferably, the DNA oligonucleotide libraries are contacted with the ultrathin cryosections on microscope slides, preferably on laser microdissection slides under conditions that have been previously established for DNA- and RNA-cryoFISH (Branco, M. R. & Pombo, A, 2006, Intermingling of chromosome territories in interphase suggests role in translocations and transcription-dependent associations. PLoS Biol. 4, e138; Xie, S.Q. et al., 2006, Splicing speckles are not reservoirs of RNA polymerase II, but contain an inactive form, phosphorylated on Serine2 residues of the C-terminal domain. Mol. Biol. Cell 17, 1723-1733; Branco, M. R., 2006, Correlative microscopy using Tokuyasu cryosections: applications for immunogold labelling and in situ hybridisation. In "Cell Imaging (Methods Express Series)", ed. D. Stephens, Scion Publishing Ltd. (Bloxham, UK), 201-217; Ferrai, C., et al., 2010, Poised transcription factories prime silent uPA genes prior to activation. PLoS Biology 8, e1000270.).

Optionally, the compartment is not sectioned before probe hybridization. Instead, the ssDNA oligonucleotide probes may be contacted with the whole compartment, e.g., a group of cells or a whole cell, which may be or may comprise the whole compartment, or a complete nucleus or other organelle. In case the compartment of the invention is a group of cells, e.g., in a tissue, the probes of the invention may hybridize to the group of cells in a suspension. The cells may be subsequently washed to remove unbound or weakly bound probes, followed by their separation into single-cell fractions by FACS. Hybridized probes may then be extracted from each individual cell for preparation of sequencing libraries. The individual libraries may next be pooled and sequenced.

Alternatively, the group of cells may be compartmentalized in oil droplets, as seen in Droplet-Seq and HyPR-Seq. Single cells can be isolated with a PCR mix (such as 1X EvaGreen Supermix) and barcoded beads, e.g., as designed in Macosko et al. (2015, Highly Parallel Genome-wide Expression Profiling of Individual Cells Using Nanoliter Droplets. Cell 161(5), 1202-1214) and provided by Chemgenes (https://www.fishersci.com/shop/products/macosko-2011-10-b/nc0927472). Cell preparation requires no prior cell lysis before PCR as it is sufficient to directly denature probes (at a temperature of 95°C) from their targets into the PCR mix and subsequently amplify them; PCR amplification takes place within the compartmentalized oil droplet. Barcoded beads individually barcode all probes from the same cell, providing single cell resolution. Oil droplets are denatured and all PCR products are sequenced. Common use Droplet machines are the BioRad QX200, Quanti3D system and the Nadia system (Dolomite Bio).

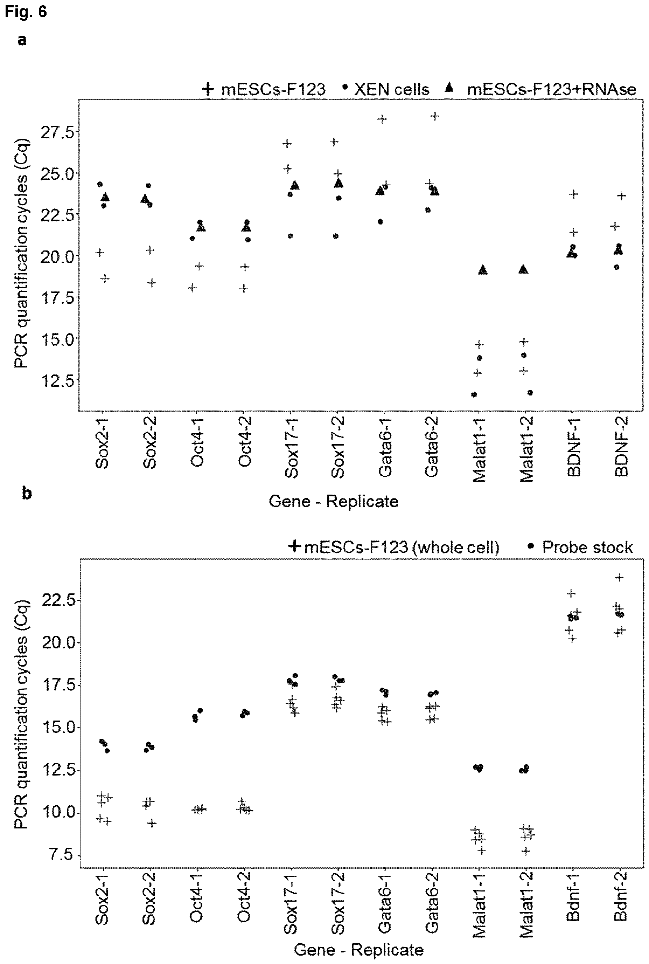

The compartment may be on a solid support, e.g., a slide, such as in a cytospin or a cell monolayer, or the compartment may be in suspension (Fig. 6b). Cells and/or compartments are preferably fixed to preserve nucleic acids, and to allow for access of the probe to the compartment of interest. Different fixation regimens can result in loss of proteins or nucleic acids from the cell or a specific compartment, and be used to favor detection of RNA in a specific compartment, for example the nucleus (Levsky, J.M. et al.,2002, Single-cell gene expression profiling. Science 297, 836-840; Pombo, A., 2003, Cellular genomics: which genes are transcribed when and where? Trends Biochem. Sci. 28, 6-9.). In some embodiments of the invention, the cells and/or compartments may be treated with a mild detergent, Triton X-100, or saponin, or Tween-20, at e.g. 0.05% up to 5%, preferable 0.1 to 0.5%, for 1-120 min, preferably 10-30 min, or other agents, such as protease, to enhance access of the primary probe to the nucleic acid within the compartment of interest.

Hybridization of ssDNA oligonucleotide probes to nucleic acids, e.g., RNA, according to step (b) may be preceded by fixation of the nucleic acid-containing compartment, wherein, optionally, the nucleic acid-containing compartment is sectioned after fixation.

Numerous fixation methods are known from the state of the art. Fixation may, for instance, involve the use of precipitating fixatives such as e.g. methanol, ethanol or acetone. Precipitating fixatives are especially suitable for fixation of frozen sections.

In a preferred embodiment, fixation of the nucleic acid-containing compartment is achieved via a crosslinking-agent such as formaldehyde or glutaraldehyde. Crosslinking may also be achieved through use of UV or ionizing radiation. Since radiation is a potent mutagen capable of destroying the integrity of nucleic acids such as RNA and DNA, crosslinking by radiation is however less preferable.

Formaldehyde is preferably used as a crosslinker, e.g., at a concentration of 0.5-8%, preferably, 1-8%, 2-8% or, most preferably, 4-8% (all w/w), e.g., in a buffered solution of 250 mM HEPES-NaOH pH 7.0-8.0, or in PBS, or cytoskeletal (CSK) buffer (Tripathi et al., 2015, RNA Fluorescence In Situ Hybridization in Cultured Mammalian Cells. In: Carmichael G. (eds) Regulatory Non-Coding RNAs. Methods in Molecular Biology (Methods and Protocols), vol 1206. Humana Press, New York, NY. https://doi.org/10.1007/978-1-4939-1369-5_11). For mammalian cells, conditions preferably include a pH 7.6-7.8, for 10 min to 24 h, e.g., for 10 min at 4% formaldehyde followed by 2 h in 8%. For example, in the case of experimental organisms, whole tissues or organisms can be crosslinked by perfusion with HEPES-buffered formaldehyde solution (e.g., 4%), or PBS-buffered formaldehyde solution, preferably for at least 30 min, followed by tissue dissection in ice cold 4% formaldehyde in 250 mM HEPES-NaOH pH 7.6 for 30 min to 1 h, followed by ice-cold 8% formaldehyde in 250 mM HEPES-NaOH pH 7.6 for 1-3 h (Moller et al., 2012, Proteomic analysis of mitotic RNA polymerase II complexes reveals novel interactors and association with proteins dysfunctional in disease. Mol. Cell. Proteomics 11(6):M111.011767; Winick-Ng et al., 2020, Cell-type specialization in the brain is encoded by specific long-range chromatin topologies, Biorxiv. https://doi.org/10.1101/2020.04.02.020990; https://www.protocols.io/view/Stellaris-RNA-FISH-Protocol-for-FrozenTissue-iwgs5v; https://www.protocols.io/view/exfish-tissue-slice-n6adhae).

Fixation of tissues or cells may occur at different strengths. The method of the invention allows for nucleic acid detection even at very strong cross-linking conditions, such as treatment with 4-8% formaldehyde, e.g., for several hours. A high level of cross-linking preserves cells more effectively, and favors retention of nucleic acid species during subsequent procedures. High cross-linking thus prepares cells for time-consuming applications or for being subjected to high mechanical stress. Surprisingly, the present invention can be applied even on highly cross-linked cells or tissues, especially when combined with sectioning.

In another embodiment, the nucleic acid-containing compartment is not fixated prior to oligonucleotide probe hybridization, wherein it optionally is vitrified. Vitrification refers to rapid freezing of a cell, tissue or whole organism to preserve cellular ultrastructure and avoid any artefacts that may potentially be introduced by the application of chemical crosslinking agents (e.g. formaldehyde).

In step (b) of the method of the invention, a nucleic acid-containing compartment, or preferably, fractions, e.g., ultrathin fractions thereof, are contacted with a plurality of single-stranded oligonucleotide probes. A probe is a section of DNA, RNA or a chemically modified oligonucleotide, e.g., comprising LNA (Locked nucleic acid) or consisting thereof, that can be used to detect the presence of a nucleic acid in a sample. In the present invention, the probe is a single-stranded DNA oligonucleotide. DNA oligonucleotides are short DNA molecules of usually 150 nt length or less. They can be manufactured as single-stranded molecules with any sequence, which can be specified by a user. Alternatively, the sequences may also be random. These oligonucleotides can bind to a target nucleic acid via sequence-specific complementary base pairing to form stable duplexes. The higher the number of complementary base pairs between the oligonucleotide probe and its target sequence, the tighter the non-covalent bonding between the two strands. Stringent washing will result in the removal of non-specific probes, retaining only strongly-paired strands bound to each other. Specificity may also be increased by the use of a chemically modified RNA molecule with increased hybridization properties (melting temperature), i.e., comprising LNA, e.g., to detect a specific single nucleotide polymorphism, or to favor detection of a low abundance RNA species in clinical samples (Domiguez and Kolodney, 2005, Wild-type blocking polymerase chain reaction for detection of single nucleotide minority mutations from clinical specimens. Oncogene 24, 6830-6834). A plurality of probes refers to any number of DNA oligonucleotides greater than one. The precise number of probes that form a so-called probe library according to the invention may vary and corresponds to the number of genes to be tested by the present invention.

In a preferred embodiment, the probes according to the invention do not comprise any molecular tags, e.g., a DNA- or RNA-tag, connected to the probe via a cleavable motif such as, e.g., a restriction enzyme site or a photo-cleavable linker. Accordingly, it is preferred that the method of the invention does not comprise a step of cleaving a molecular tag, such as a DNA- or RNA-tag, off the probe, e.g., avoiding the need for UV treatment or restriction enzyme treatment.

In a preferred embodiment, the nucleic acid to be detected by the probes used in the method of the invention, i.e., the target nucleic acid, is RNA.

The herein described method neither sequences an RNA molecule itself nor a cDNA generated thereof, nor a cleavable (e.g., DNA-) label or tag, but rather identifies the RNA of interest by directly sequencing or amplifying the ssDNA oligonucleotide probes complementarily bound to it. Therefore, the method of the invention preferably does not comprise a cDNA generation step. The contacting of step (b) is carried out under conditions that allow for binding (or hybridizing) of the probes to RNA molecules within said compartment. Thus, the probes hybridize to RNA molecules within said compartment. The conditions may be chosen not to allow for hybridization of the probes to DNA that may optionally also be comprised in the compartment, e.g., a DNase can be used to pre-treat the compartment prior to ssDNA probe hybridization to remove dsDNA and ssDNA (Pombo, A., et al., 1994, Adenovirus replication and transcription sites are spatially separated in the nucleus of infected cells. EMBO J. 13(12), 5075-5085). Alternatively, specific dsDNases may be used post-probe hybridization to remove dsDNA. A suitable dsDNase may be obtained commercially, e.g. from ThermoFisher (Cat.No.: EN0771).

Exemplary conditions for hybridizing the probe to RNA are at 30-65°C, e.g., 37-55°C, e.g., in a saline-sodium citrate (SSC) buffer containing 10-50%, preferably 30%, formamide and 5-20%, preferably 10%, dextran sulfate. Optionally, the buffer may be supplemented with a suitable inhibitor of ribonucleases such as ribonucleoside vanadyl complexes (RVC) or, in case the hybridization buffer does not comprise formamide, RNaseOUT^{™}. The buffer may further comprise tRNA (from e.g. yeast) as a non-specific blocker, e.g., at a concentration of about 1 mg/mL. Hybridization may take place, for example, for at least 15 min to a week, preferably, at least 30 min, e.g., at least 45 min, at least 1 h, at least 2 h or at least 5h. It may also last longer, e.g. overnight, two overnights or three overnights. Suitable conditions are also described in the examples below. Of note, temperatures and formamide concentrations do not only affect probe hybridization but also modulate the stringency of stringent washes used to remove unbound or partly hybridized probes. Accordingly, the above chosen conditions greatly contribute to the sensitivity and specificity of the method of the invention.

In another embodiment, the nucleic acid to be detected by the method of the invention may be DNA, preferably single-stranded DNA.

Accordingly, the contacting step (b) is to be carried out under conditions that allow for hybridizing of the probes to DNA molecules within said compartment. For instance, if the DNA to be detected is dsDNA, it has to be denatured to obtain ssDNA accessible to probe hybridization. Optionally, an RNase may be used to pre-treat the compartment prior to or after probe hybridization to remove RNAs from the sample. Probe hybridization may occur under the same conditions as described above for detection of RNAs.

For accurate DNA detection, the method of the invention may be further adapted to allow for reliable differentiation between double-stranded DNA-probe complexes and fragments of naturally occurring dsDNA (e.g. genomic or plasmid DNA). For instance, the oligonucleotide probes may be modified with barcode sequences or unique molecular tags to facilitate their identification, as explained herein. Alternatively, genomic DNA modifications such as cytosine methylation may be taken into account to exclude genomic DNA from analysis.

In yet another embodiment, the method of the invention allows for simultaneous detection of both RNA and DNA in a compartment. To detect both RNA and dsDNA, e.g., probes can be added to hybridize to RNA first, without denaturation of dsDNA, and then another set of probes, e.g, with different barcode sequences or tags, may be added to hybridize to DNA after denaturation thereof. RNA and ssDNA can also be detected simultaneously, as described above for detection of RNA, but avoiding conditions that destroy ssDNA.

In a preferred embodiment, the method of the invention does not comprise an RNA isolation step. Advantageously, no nucleic acid isolation is carried out before step b). The herein described method utilizes ssDNA oligonucleotide probes that, preferably, hybridize to a nucleic acid *in situ,* i.e., within the compartment in which the nucleic acid naturally occurs. Therefore, the method of the invention is suitable for localizing a detected nucleic acid within a compartment.

In another embodiment, the method of the invention may also be used to detect nucleic acid that has been isolated from the compartment. The nucleic acid may, e.g., be detected *in vitro,* e.g. in a suitable buffer within a test tube. In such a scenario, the ssDNA oligonucleotide probes may be directly added to the isolated nucleic acid inside the test tube to initiate hybridization. Alternatively, probe hybridization may take place *in situ,* prior to isolating the nucleic acid and all bound probes from the nucleic acid-containing compartment.

The ssDNA oligonucleotide probes used in the method of the invention may be generated by any method known in the art. E.g., each probe may be synthesized separately (Femino, A.M., et al., 1998, Visualization of Single RNA Transcripts in Situ. Science, 280(5363), 585-590. More recently, high numbers of probes were prepared by massive parallel synthesis on a solid substrate such as a microchip, before, optionally, being amplified and released into solution (Beliveau et al., 2012; Beliveau et al., 2017, In situ super-resolution imaging of genomic DNA with OligoSTORM and OligoDNA-PAINT. Methods Mol Biol. 1663, 231-252; https://oligopaints.hms.harvard.edu/protocols).

The ssDNA oligonucleotide probes according to the invention may have a length of about 55-150 nucleotides (nt), preferably 70-120 nt, or about 80 nt. In the examples below, probes have a length of 115 nt. In a preferred embodiment, probe length may also be about 75-85 nt.

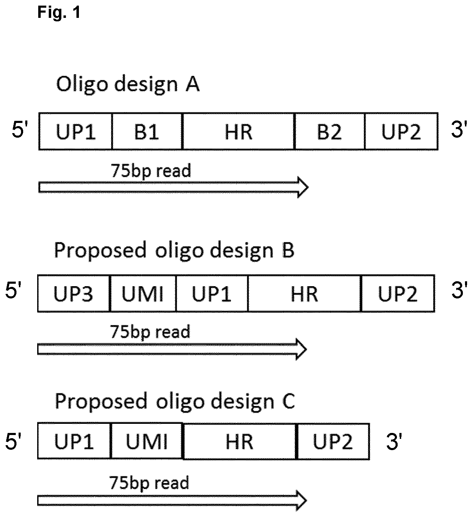

Each probe typically comprises a target region that is complementary to a nucleotide sequence of a target nucleic acid (e.g., a target RNA) flanked by a pair of primer regions, e.g., universal primer regions (Fig. 1; probe structure). The nucleotide sequence of this central target region specifically hybridizes to an individual nucleic acid. Hybridization refers to a process wherein two single-stranded nucleic acid molecules anneal to each other via complementary base pairing, i.e. the nitrogenous base adenine present in one nucleic acid strand pairs and forms hydrogen bonds with thymine (DNA) or uracil (RNA) in the opposing strand, whereas cytosine pairs with guanine. Importantly, the targeting region of the probe according to the invention is designed to display high stringency towards its respective target nucleic acid to ensure specific binding, i.e., it cannot anneal to different nucleic acids. Preferably, the target region exhibits 100% complementarity to a sequence within the nucleic acid of interest. Accordingly, the herein described method has the potential to differentially detect nucleic acid molecules that differ even in a single nucleotide. A possible method for designing highly sensitive oligonucleotide probes capable of distinguishing different alleles by detecting SNPs is detailed in Beliveau et al., 2014 and relies on publicly available SNP collection databases that exist for different species. The target region of a probe is designed to possess a stability that allows a duplex formed by the probe and its target nucleic acid to withstand stringent washes at temperatures between 37°C and 65°C to remove partially hybridized probes, before denaturation of the specifically hybridized probe, i.e., before the probe is removed from its target nucleic acid. In the example below, stringent washing was performed in a buffer comprising 40% formamide at 47°C, in the presence of dextran. The target region may have a length of, e.g., 20 to 50 nt, preferably, 30 to 40 nt, e.g., 30, 31, 32, 33, 34, 35, 36, 37, 38, 39 or 40 nt. In a particular preferred embodiment, the target region has a length of about 35 nt. It may also be longer than 40 nt, e.g., 40-50 nt or 40-45 nt. Increasing the length of the target region increases probe specificity. The target region may however also have a length shorter than 30 nt, e.g., to hybridize to small target nucleic acids. For instance, mature miRNAs have a length of merely 20-25 nt. Accordingly, the length of a probe's target region may thus correspond to the length of the targeted miRNA. The target region of a probe may have a minimum length of 20 nt to still ensure specific and selective detection of a target nucleic acid. Besides probe length, hybridization stringency also depends on the precise nucleotide composition of the probe. Higher GC (guanine/cytosine)-contents are usually associated with a higher stringency.

In other embodiments, the target region or the probe(s) exhibits less than 100% complementarity to a nucleic acid of interest, e.g., 85-99% or 90-95% complementarity. Imperfect complementary may e.g. be desired for applications where the exact genomic sequence of a target nucleic acid is not known with 100% confidence. Particularly in humans, SNPs between different alleles may be unknown. Probes that allow for a given amount of mispairing may thus be utilized to assess combined gene expression from two different alleles.

In an optional embodiment, the target region of a probe may not be designed to hybridize to a defined target nucleic acid. Instead, the target region may consist of a stretch of randomly assembled nucleotides. Such a "random" oligonucleotide probe may be added to the compartment with the intention of hybridizing to yet unknown RNA or DNA molecules.

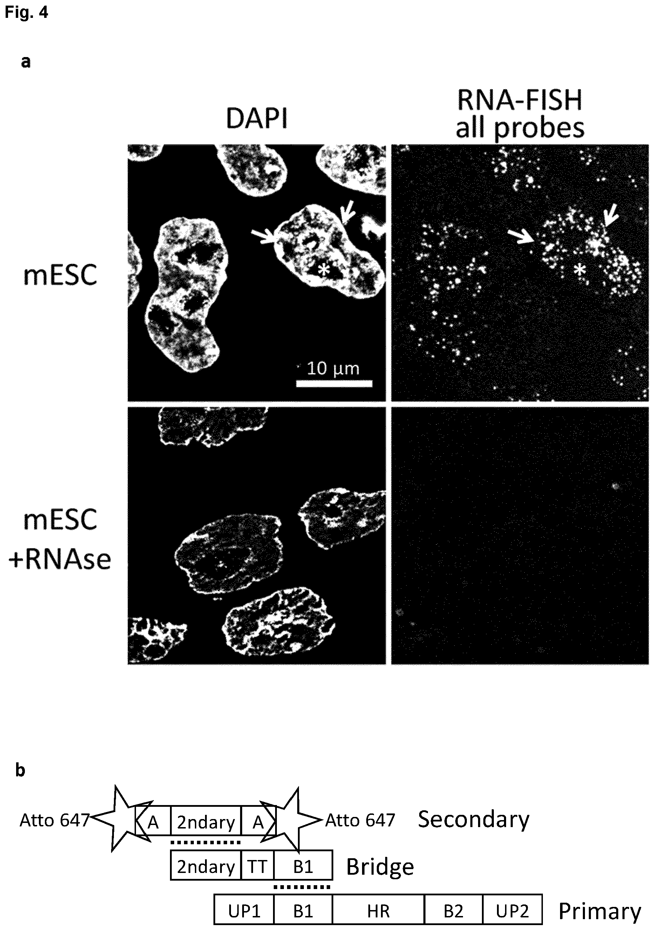

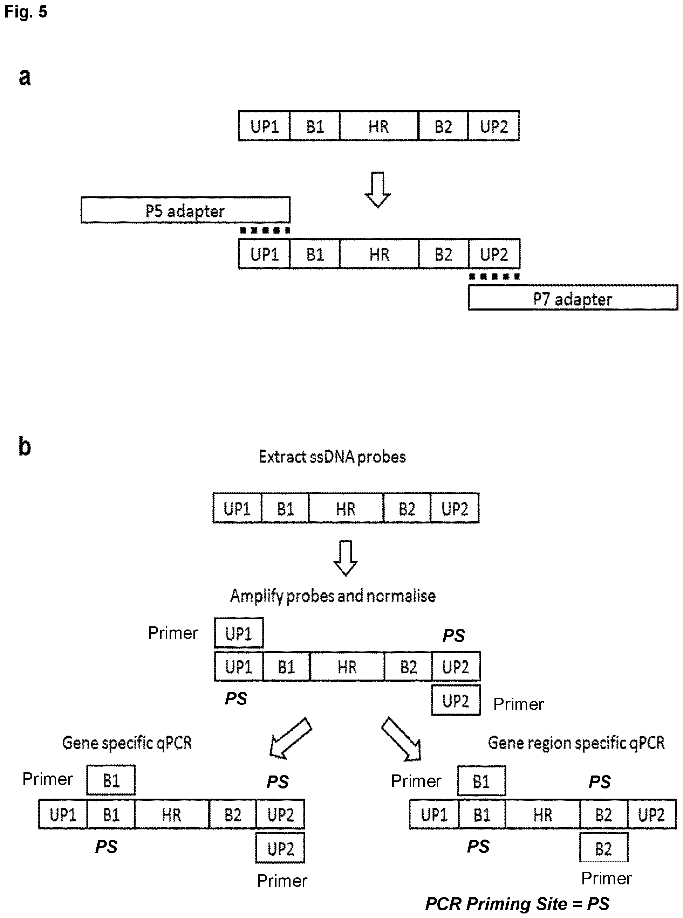

The universal primer regions on each side of the target region enable amplification of probes and can be appended to sequencing adaptors (Fig. 5a). Optionally, the universal primer regions may be used as target sites for fluorescent *in situ* hybridization (FISH) probes to enable straightforward microscopic validation of successful probe hybridization, and during optimizations (Fig. 4). Each universal primer has a length of 15-30, preferably 20-25, e.g., 21-23 nt. Optionally, the universal primer has a length of 22 nt.

Optionally, the single-stranded DNA oligonucleotide probes further comprise a unique molecular identifier (UMI). A UMI is a randomly assembled short nucleotide sequence that serves as a unique molecular tag. Each UMI individually labels a particular probe and thus facilitates reliable probe identification while effectively reducing errors and quantitative bias introduced by amplification. Labeling a probe of the invention with a UMI is preferred for applications wherein the nucleic acid, e.g., RNA, inside the compartment is to be quantified. UMIs can furthermore facilitate the identification of, e.g., rare transcript variants, especially when dealing with low overall quantities of RNA in a sample.

Optionally, the ssDNA oligonucleotide probe may further comprise at least one (e.g., at least two) identifying barcode sequence. A barcode differs from a UMI in that it does not uniquely identify only an individual probe, but labels a collection of distinct probes that can be grouped according to common characteristics (Beliveau et al., 2012). For example, a barcode sequence may label all probes that target the same specific gene of interest. Alternatively, and/or in addition, it may be shared by all probes targeting a same genic region of a gene, e.g. an exon and intron or an exon/intron junction. Barcodes may also summarize probes for genes of a particular function or sharing a common pathway. The barcode sequences may in addition serve as binding regions for primers during PCR-based quantification.

Optionally, the single-stranded DNA oligonucleotide probes do not comprise an UMI. Optionally, the single-stranded DNA oligonucleotide probes do not comprise a barcode sequence, and in one embodiment, they comprise neither an UMI nor a barcode sequence. These components of the probes are not required, and, as they also need to be sequenced or amplified in the method of the invention, may introduce a bias that may be advantageous to avoid. The barcodes further have potential to cause off target annealing events, i.e. anneal to an RNA not intended for targeting, reducing specificity of the assay which can lead to noise in the data. This may be more problematic when they are adjacent to a UMI or primer regions or homology sites, further increasing chances for off target binding, or by a higher number of DNA barcodes used. Thus, it is preferred that the probes do not comprise a barcode sequence.

Although it may also be of interest to perform the method of the invention with a single type of probe, e.g., to find out if a specific RNA is expressed in a cell, typically, the plurality of probes is a plurality of different probes, i.e., the single-stranded DNA oligonucleotide probes used in the method according to the invention specifically hybridize to a plurality of target nucleic acids, e.g., target RNAs, present in the compartment, or to different regions of one target nucleic acid. For instance, probes may be used for targeting a set of RNAs transcribed from genes that serve as specific cell type and/or differentiation markers, or to detect a viral transcript. The probes may target, e.g., transcripts of well-known stem cell markers such as Oct4 or Sox2. Probes may also detect transcripts of marker genes that signify and track differentiation of stem cells into a distinct cell type or lineage. Other subsets of genes that may be targeted by a plurality of probes include e.g., sets of inflammatory cytokines, members of a particular signaling cascade or particular cancer markers and other disease markers. Probes may also be specifically designed to detect cell cycle marker genes such as cyclin E or B to categorize different compartments or fractions thereof based on their stage in the cell cycle. Further probes targeting known housekeeping genes common to all or most analyzed cell types and non-responsive to environmental changes may be included as controls to e.g. normalize analyses of differential gene expression.

A typical probe library that may be used may target several hundreds of nucleic acids, e.g., mRNAs transcribed from genes such as marker genes. Such a probe library may be suitable for multiple applications. For instance, a probe library may target at least two nucleic acids, e.g., 2-100000, 3-50000, 4-25000, 5-10000, 10-5000, 15-2000, 20-1000, 25-500, 30-250, 40-200, 50-100 nucleic acids, e.g., RNAs such as mRNAs. It may also target at least 50, at least 100, at least 200, at least 300, at least 400, at least 500, at least 600, at least 700, at least 800, at least 900 or at least 1000 nucleic acids, e.g., RNAs such as mRNAs. In some embodiments, a probe library may also target less than 50 nucleic acids, e.g., RNAs. For instance, if probes are to be detected by PCR-based probe amplification rather than massive parallel sequencing, a probe library targeting up to 5, up to 10, up to 15, up to 20, up to 25, up to 50, up to 75, up to 100, up to 200, up to 300, up to 400, or up to 500 nucleic acids may be prepared. However, in a preferred embodiment, probes from a common probe library, or the complete common library may be used, independent on whether the probes are to be identified via sequencing or amplification.

To maximize the amount of information obtainable from the method of the invention, a single nucleic acid, e.g., an mRNA transcript may, e.g., be targeted at different positions by multiple probes. Multiple probes may for instance cover several exons, introns as well as exon/intron or exon/exon junctions of a single gene to detect distinct splicing variants. Alternatively, probes may be specifically designed to target or recognize allelic variants of a transcript from a gene that differ e.g., due to nucleotide deletions, insertions, inversions or substitutions. The probes used for the method may exhibit sufficient specificity to faithfully differentiate between transcripts that differ even in a single SNP. Gene fusions may be detected by designing probes targeting known fusion junctions, or by comparing the detection levels of probes that bind to the 3' or the 5' end of a transcript from a gene, respectively.

In one embodiment, probes may cover a nucleic acid, e.g. a transcript from a gene to full extent, including every exon and intron. Accordingly, the number of probes able to bind a single transcript may depend on the length of the encoding gene. For instance, a transcript with a length of 2000 nt may be targeted by more probes than an RNA with a length of 500 nt. Designing probes covering the entire length of a transcript from a gene of interest will provide a maximum amount of information for that gene or transcript, in particular the presence of splice variants, isoforms or fusion products. For example, a probe for every exon and intron, or, if applicable, every splice-variant of the gene may be used. However, in some experiments, it may suffice to determine whether any given gene is actively transcribed or not, especially when a large number of genes is under consideration. Therefore, to reduce costs and workload, an RNA molecule may be targeted only by a few probes or even not more than a single specific probe. This is possible, since the presence of an RNA is to be determined by the method of the invention via sequencing or amplification of the probe. By contrast, FISH-based methods that detect RNAs by microscopy usually require hybridization of multiple probes to a transcript to produce a detectable FISH-signal. Similarly, the FISH-based HyPR-seq requires the hybridization of at least one pair of initiator probes to an RNA of interest followed by the sequential annealing of a metastable hairpin oligo and a "readout oligo".

Therefore, nucleic acids, e.g. transcripts from genes, may be targeted by approximately 1 - 1000, 50 - 700, 100 - 500, 150 - 400, 200 - 300, but also as few as 1 - 100, 1 - 50, 1 - 20, 1 - 10, 1 - 5 or even 1 - 3 oligonucleotide probes. Probe concentration sufficient for effective RNA detection according to the invention depends on the size and complexity of the initial probe library, but also on the compartment under investigation and how it is processed prior to probe hybridization. For effective RNA detection on cellular cryosections of about 200 nm thickness, each oligonucleotide probe may for example be used at a concentration of about 0.1 to about 1 µM, preferably 0.2 - 0.8 µM, 0.3 - 0.7 µM, or 0.4 - 0.6 µM in a suitable buffer solution (e.g. water). In a particularly preferred embodiment, the probe may have a concentration of 0.5 µM. Preferably, approximately 0.1nM per unique probe in the library.

Preferably, the single-stranded DNA oligonucleotide probes specifically hybridize to a target RNA selected from the group comprising RNAs involved in protein biosynthesis comprising mRNAs, rRNAs, snRNAs, snoRNAs and tRNAs, small regulatory RNAs comprising miRNAs, siRNAs, shRNA and piRNAs, long regulatory RNAs comprising eRNAs and IncRNAs, circRNAs, tracrRNAs, crRNAs, retrotransposons, viral RNAs, satellites, TERC, vtRNAs, DDRNAs, PROMPTs, or a combination thereof.

In principle, the present invention thus enables detection and identification of every RNA molecule found in a compartment.

In a preferred embodiment, the target RNA is an mRNA. mRNAs transmit genetic information from the DNA blueprint to ribosomes, where the encoded information is translated into a polypeptide chain. Detection of mRNAs in a compartment thus provides valuable information on the expression of protein-coding genes. In one embodiment, the single-stranded DNA oligonucleotide probes of the invention may form a library that specifically hybridizes to substantially all mRNAs present in the compartment. Using such a probe library, the method is therefore not limited to detecting only a comparably small subsets of e.g. marker mRNAs, but rather enables detection of the entire protein-coding transcriptome. The method of the invention thus also provides a means for studying, e.g., differences in global gene expression in response to changes in environmental conditions, ageing or upon altered (i.e. compromised or enhanced) cell signaling.

In eukaryotic cells, mRNAs are transcribed from DNA inside the cell nucleus by an RNA polymerase as long precursor transcripts known as precursor mRNAs (pre-mRNAs). Before being transported into the cytoplasm for their translation into protein, pre-mRNAs undergo successive processing steps in the nucleus. During one of these steps, pre-mRNAs are subjected to a process termed splicing, wherein non-coding intergenic regions (introns) are excised from the nascent transcript and the remaining coding regions (exons) are fused together to obtain a mature mRNA. A single pre-mRNA may be spliced in multiple alternative ways, dependent on the choice of segments that are considered as introns or exons by the splicing machinery (spliceosome). For instance, exons can be extended or skipped and introns can be retained in the final transcript. Alternative splicing results in distinct mature mRNA variants that may give rise to a range of unique proteins with different functional properties. Probes used in the context of the invention may detect pre-mRNAs and/or such splice variants. Probes may further be designed to detect gene isoforms, i.e. mRNAs that are produced from the same gene or genetic locus but differ from each other as they were transcribed e.g. from distinct transcription start sites (TSS).

mRNAs however represent just a minor fraction of all RNAs present in a cell. Only an estimated 2% of the entire human genome encodes for proteins. The other 98% of RNA transcripts found in mammals are considered to be non-coding (Dhanoa, J. K., Sethi, R. S., Verma, R., Arora, J. S., & Mukhopadhyay, C. S., 2018, Long non-coding RNA: its evolutionary relics and biological implications in mammals: a review. Journal of animal science and technology, 60, 25). Therefore, ssDNA oligonucleotides used for the method of the invention may alternatively or additionally hybridize and detect non-coding RNAs. For example, probes may detect transfer RNAs (tRNAs). tRNAs transport amino acids to ribosomes and play a central role during the translation of an mRNA into a polypeptide chain. Probes may also be specifically designed to detect ribosomal RNAs (rRNAs). rRNAs represent the most commonly found class of RNAs in a cell and associate with a set of proteins to form ribosomes. rRNAs fulfil not only a structural function, but also exert enzymatic activity by catalyzing the formation of peptide bonds between amino acids to form a polypeptide chain. rRNAs thus belong to the class of ribozymes. Due to their ubiquitous abundance, rRNAs are mostly excluded from classical RNA-Seq experiments, either via their selective depletion or by exclusively pre-selecting polyadenylated transcripts prior to sequencing. However, comparisons of rRNA sequences among species provide useful information on phylogenetic relationships and on species diversity.

Another example for ribozymes detectable by probes in the method according to the invention are small nuclear RNAs (snRNA) that together with a group of proteins form the spliceosome. snRNAs catalyze the removal of introns from pre-mRNAs during the splicing process. rRNA, snRNA and tRNA processing is modulated within a sub-region of the nucleus, the nucleolus, by the family of small nucleolar RNAs (snoRNAs).

Regulatory RNAs are non-coding RNAs that modulate various biological processes. Among this group of RNAs, small regulatory RNAs are especially well studied and thus of great interest for transcriptome studies. Small regulatory RNAs possess a size of 40 nt or less, and usually post-transcriptionally regulate gene expression. Among these small RNAs, microRNAs (miRNAs), small interfering RNAs (siRNAs) and Piwi-interacting RNAs (piRNA) are best understood. It is estimated that hundreds of distinct and evolutionary conserved miRNAs modulate the expression of more than 60% of all human protein-coding genes within virtually every biomolecular pathway (Catalanotto, C., Cogoni, C., & Zardo, G., 2016, MicroRNA in Control of Gene Expression: An Overview of Nuclear Functions. International journal of molecular sciences, 17(10), 1712). Especially due to their special role in the regulation of cell death and proliferation, microRNAs can act both as tumor suppressors or oncogenes. They are thus commonly used as tumor markers. Probes designed to specifically hybridize to such cancer-associated miRNAs may thus reliably identify cancer cells in tissues. miRNAs are generated from long precursor transcripts that form hairpin structures and are processed through endonucleolytic cleavage. miRNAs silence gene expression post-transcriptionally through a molecular mechanism known as RNA interference (RNAi), wherein they guide a protein complex to a target mRNA via complementary base-pairing to initiate mRNA degradation or translational inhibition. Small interfering RNAs (siRNAs) rely on a similar biogenesis pathway as miRNAs, but originate from double-stranded RNA molecules. Similar to miRNAs, they use RNAi to prevent target mRNAs from being translated into protein. The probes of the method according to the invention may specifically detect, e.g., the processed mature versions of these different small RNA species. Additionally or alternatively, probes may be designed to detect their longer primary or precursor transcripts. Short hairpin RNAs (shRNA) are artificially produced molecules that are introduced exogenously by researchers into cells or organisms to decrease expression of a gene of interest through the RNAi machinery (gene knockdown). The method of the invention may allow scientists to verify the effectiveness of shRNA introduction and gene knockdown by assessing the levels of shRNAs and their target mRNAs, e.g., in their model organism or cell. The slightly longer Piwi-interacting RNAs (piRNAs) are small non-coding RNAs. They are best known for silencing transposable elements.

In addition to short regulatory RNAs such as miRNAs, siRNAs or piRNAs, the subset of non-coding regulatory RNAs further comprises the class of long non-coding RNAs (IncRNAs) and enhancer RNAs. IncRNAs are a highly expressed in humans, are of at least 200 nt length and are frequently processed in the same manner as mRNAs. However, IncRNAs differ from mRNAs in that they usually lack an open reading frame (ORF). LncRNAs modulate various cellular processes such as transcription, post-transcriptional regulation, RNAi, splicing, translation or epigenetic regulation. However, the biological relevance of many IncRNA is currently unclear (Dhanoa et al., 2018). For instance, many IncRNAs may in fact encode peptides of unknown function (van Heesch et al., 2019, The Translational Landscape of the Human Heart. Cell 178(1), 242-260). Analysing IncRNA with the method of the invention may provide new information on their expression, localization and, ultimately, function.

eRNAs have a length of 50-2000 nt and are transcribed from the DNA sequence of enhancer regions. Their expression correlates with the activity of their corresponding enhancer and are thus suitable markers for differentiating between active and quiescent enhancers (Arnold et al., 2020, Diversity and Emerging Roles of Enhancer RNA in Regulation of Gene Expression and Cell Fate. Front. Cell Dev. Biol.). eRNAs may regulate mRNA transcription by promoting enhancer-promoter interactions, chromatin modifications or regulation of the transcriptional machinery.

Circular RNAs (circRNAs) are single-stranded, loop-forming RNA molecules, often with an unclear molecular function. Some circRNAs are believed to act as miRNA sponges (Kristensen et al., 2019, The biogenesis, biology and characterization of circular RNAs. Nat Rev Genet. 20(11), 675-691), while others seem to encode proteins (van Heesch et al., 2019). Importantly, expression of specific circRNAs in the human brain has been associated with the development and progression of neurodegenerative disorders, in particular Alzheimer's Disease (AD). They are thus considered as potential biomarkers for AD diagnosis (Akhter, 2018, Circular RNA and Alzheimer's Disease. Adv Exp Med Biol. 2018;1087:239-243).

The present invention may also be suitable for detecting foreign, pathogenic RNA in a cell, e.g. viral RNA genomes and satellites, or parts thereof. It may further be suitable for detecting bacterial RNAs involved in anti-viral immune defenses such as trans-activating RNAs (tracrRNAs) or CRISPR-RNAs (crRNAs) and their modified versions used for CRISPR/Cas-mediated genetic engineering. TERC (short for telomerase RNA component) is a non-coding RNA found in eukaryotes and serves as a template for the extension of telomeres by the enzyme telomerase (Feng et al., 1995, The RNA component of human telomerase. Science 269(5228), 1236-1241). DDRNAs are short non-coding RNAs generated at sites of DNA double-strand breaks and are required for initiating a DNA damage response in cells (Michelini, F., Pitchiaya, S., Vitelli, V. et al., 2017, Damage-induced IncRNAs control the DNA damage response through interaction with DDRNAs at individual double-strand breaks. Nat Cell Biol 19, 1400-1411). Retrotransposons are DNA sequences inside a genome that are transcribed into RNA and are later converted back into DNA by reverse transcription followed by their insertion at a different genomic position. Vault RNAs (vtRNAs) are part of ribonucleoprotein particles known as vaults with possible functions in intracellular, in particular nucleocytoplasmic transport processes. Promoter upstream transcripts (PROMPTs) are non-coding RNAs transcribed in the reverse orientation of most active protein-coding genes at around 1-1.5 kb upstream of the transcription start site. PROMPTS are usually rapidly degraded by the RNA exosome (Lloret-Llinares et al., 2016, Relationships between PROMPT and gene expression. RNA Biol. 13(1), 6-14). In summary, while recent years have witnessed the discovery of numerous novel species of RNAs, their functions are often not fully understood. The method according to the invention may be vital for obtaining novel information on these RNA species through their selective recognition by probes in the method of the present invention.

Alternatively, the single-stranded DNA oligonucleotide probes may specifically hybridize to a target DNA selected from the group comprising double-stranded DNA comprising chromosomal DNA, mitochondrial DNA, chloroplast DNA, bacterial DNA, plasmid DNA, viral dsDNA or double-stranded DNA transposable elements (transposons).

Plasmids are small circular extrachromosomal DNA molecules that are physically separated from chromosomal DNA. Plasmids are usually found in bacteria and often carry genes that are beneficial for the bacterium's survival, e.g. antibiotic resistances. Artificially generated plasmids are frequently used as vectors to genetically modify cells or whole animals or for conducting molecular cloning.

Viruses of group I of the Baltimore classification system possess dsDNA as their genetic material. In some viruses, the viral dsDNA genome may take a circular shape (Baculoviridiae, Papovaviridiae) whereas in others, the viral DNA is linear (Adenoviridae, Herpesviridae).

DNA transposons are DNA sequences that may change their position within the genome. Using a cut and paste-like mechanism, dsDNA is moved and integrated into new genetic locations by an enzyme called transposase.

To enable hybridization of the oligonucleotide probes according to the invention, the dsDNA must first be denatured to a single-stranded state. Denaturation of naturally occurring dsDNA may be preferably achieved thermically, i.e. by elevating the temperature of a DNA-containing sample to a point at which the hydrogen bonds between the nitrogenous bases of the DNA break apart. The precise denaturation temperature depends on the length of the dsDNA molecule and its GC-content. Alternatively, DNA may be denatured chemically, e.g. by exposing it to NaOH or high concentrations of salt, or treated with DNAse I to expose ssDNA.

Before allowing the two ssDNA strands to renature into their original double-stranded form, the ssDNA oligonucleotide probes need to be added to the sample where they bind to their respective single-stranded DNA regions of interest.

In yet another embodiment, the single-stranded DNA oligonucleotide probes may be designed to specifically hybridize to a target DNA selected from the group comprising single-stranded DNA species comprising viral ssDNA genomes, helitrons as well as transiently exposed stretches of single-stranded genomic DNA present in e.g. R-loops or at sites of DNA damage. Viruses that are grouped into class II of to the Baltimore virus classification system possess a ssDNA genome that takes a circular shape. The probes according to the invention may be designed to specifically bind to and detect the viral ssDNA genome in a compartment.

Similarly, the probes may also detect specific DNA transposons known as helitrons that switch positions in a genome by a rolling cycle mechanism that involves the generation of a circular ssDNA intermediate.

The term R-loop describes a three-stranded nucleic acid structure that consists of a DNA:RNA hybrid and a non-template ssDNA. R-loops usually develop at sites of active transcription when a newly transcribed RNA threads back to hybridize with the template guide DNA strand, thereby displacing the non-template passenger DNA strand (Allison and Wang, 2019, R-loops: formation, function, and relevance to cell stress. Cell Stress 3(2), 38-47). Traditionally, R-loops are detected with sequence-independent but structure-specific antibodies followed by DNA sequencing in a method called DRIP-Seq. In the method of the present invention, specifically designed ssDNA oligonucleotide probes may e.g. hybridize to the displaced non-template DNA strand.

Stretches of ssDNA may also transiently arise under other circumstances, e.g., at sites of stalled replication forks due to DNA damage.
The oligonucleotide probes of the invention may also detect fragmented or even highly degraded DNA molecules such as ancient DNA obtained from archaeological sites or prehistoric animals.

In summary, the present invention thus also enables scientists to detect any DNA molecule of interest in a DNA-containing compartment, provided that the DNA is present in a single-stranded state prior to probe hybridization.

Accordingly, in one embodiment, the library of single-stranded DNA oligonucleotide probes may detect substantially all RNA or all DNA, or a combination thereof, as described herein, present in the compartment. A past bioinformatic analysis suggested that more than 90% of the entire human genome and 100% of the *C. elegans* genome could be covered at a density of approximately 10 probes per kb by specifically designed unique DNA oligonucleotide probes known as oligopaints that resemble the probes used in the present invention (Beliveau et al., 2012). Therefore, the present invention has the potential to selectively detect RNAs that cover up to 75%, up to 80%, up to 85%, up to 90%, up to 95%, or up to 99% of the genome present in a cell. In certain embodiments, the probe library according to the method of the invention may cover at least 99%, preferably, 100% of the RNA or DNA, or a combination thereof present in a compartment, e.g., of a tested genome. It may then detect substantially all RNA or all DNA, or a combination thereof, present in the compartment.

The method of the invention may also effectively contribute to the detection of nucleic acid modifications. Probes may for instance be designed to detect rare edited RNA molecules. RNA editing relates to a process where the nucleotide sequence of RNAs is altered after transcription, e.g., via deletion, insertion or substitutions of nucleotides. RNA editing may affect the activity, stability and localization of an RNA. Other nucleic acid modifications such as DNA or RNA methylation or base isomerizations may be detected with specific antibodies. The detection of nucleic acids with the method of the invention in combination with the identification of said nucleic acid modifications could allow the skilled person to relate the abundance of a particular nucleic acid to its modification at a given spatial position.

In an optional embodiment of the invention, specific nucleic acids that are not of interest for an experiment may be excluded from analysis by adding "cold oligonucleotides" to the nucleic acid-containing compartment prior to or simultaneously with the ssDNA oligonucleotide probes. The term "cold oligonucleotides" refers to unlabeled oligonucleotides with the function of competitively "blanking out" undesired nucleic acids. For instance, the use of the probes of the invention may be associated with the addition of oligonucleotides that lack flanking primer regions and specifically hybridize to highly abundant nucleic acids (e.g. rRNA or 7SK snRNA), but which are not amplified during the steps that follow hybridization. As a result, probes may no longer access and bind to these non-target nucleic acids. As the cold oligonucleotides lack a primer region, they are not amplified by PCR in the following step and consequentially will not be identified during e.g. sequencing.

Probes or, optionally, cold oligonucleotides that do not hybridize, or that hybridize to only part of the homologous sequences of the target nucleic acid molecules are removed from the compartment in step (c) during the method of the invention. Probe removal may involve stringent washes with a suitable solvent. Unhybridized probes may be washed from the compartment with a suitable buffer at any temperature from room temperature (20-25°C) to about 75°C. In a preferred embodiment, unhybridized probes are washed from the compartment with about 40% formamide at a temperature of 45-49°C, preferably, at 47°C.

Stringent washes may also be performed using other buffers commonly used during hybridization experiments such as saline-sodium citrate (SSC) or phosphate-buffered saline (PBS) buffer. The buffer may contain detergents, e.g. 0.1% Tween-20. Removal of unbound probes may involve at least a single washing step. It may also involve more than one washing step, e.g. two, three, four or five steps, using washing buffers of the same or, preferably, varying concentrations, e.g., of increasing stringency. In an alternative embodiment, unhybridized probes may be removed from the compartment using an endonuclease that specifically degrades free ssDNA oligonucleotides, e.g., exonuclease I.

Optionally, hybridization of ssDNA oligonucleotide probes to RNA may be verified microscopically prior to sequencing-based or PCR-based probe identification by targeting hybridized probes with a second, fluorescently-labeled FISH probe. The FISH probe may complementarily bind to a barcode region of the ssDNA oligonucleotide probe and may be visualized by a fluorescent microscope or another device suitable for detection of fluorescent dyes (Fig. 4a and Fig. 4b). However, advantageously, an imaging-based analysis of the presence of probes is not required (and, preferably, not carried out) in the method of the invention.

After removal of unhybridized ssDNA oligonucleotide probes in step (c), and before sequencing or amplification, the oligonucleotide probes that have hybridized to nucleic acid molecules within the compartment are typically extracted from said compartment. Standard methods for DNA isolation may be used.

Then, an amplification of the hybridized single-stranded DNA oligonucleotide probes may be carried out. Firstly, hybridized probes may be released from the nucleic acids in the compartment, e.g., RNA, at temperatures of about 85°C, preferably about 95°C. Alternatively, denaturation of the nucleic acid-oligonucleotide probe-complex may be achieved via alkaline hydrolysis using, e.g., NaOH. In case a probe hybridized to an RNA molecule, probe release may occur upon adding an RNA degrading enzyme, e.g. RNase H. Amplification of probes may then be achieved, typically, by polymerase chain reaction (PCR) using primers that specifically anneal to each probe. Primers may for instance be designed to hybridize to the universal primer region of the probe and/or to the target region. Preferably, the primers anneal directly to the probe that was complementarily hybridized to the nucleic acid of interest and not to any molecular tag that may have been attached to said probe via a chemical linker (e.g., a photo-cleavable linker).

The amplification primers may be designed to comprise a sequencing adaptor e.g., an adaptor sequence provided for Illumina-based NGS, at their 5' end. The sequencing adaptors may thus be added to the 5' ends of the amplified probes during the PCR reaction. In another embodiment, a probe may first be ligated to a sequencing adaptor, before its amplification. In such a case, amplification primers may be specifically designed to anneal to these sequencing adaptors to enable amplification of a readily usable sequencing library. Taq polymerase or, to minimize errors introduced by the polymerase, high-fidelity polymerases commonly known to the skilled person may be used, e.g., Q5^{®}, Phusion^{®}, Platinum^{®} or AccuPrime^{®}. In an alternative embodiment, probes are not amplified priorto their identification to prevent later problems with their identification due to replication bias or inaccurate amplification.

T7 mediated linear amplification may also be used as a means for probe amplification.

PCR amplification may preferably be followed by removal of excess primers that otherwise might interfere with probe sequencing. Primer removal may for instance be achieved by contacting the sample with a suitable exonuclease (e.g., exonuclease I) that effectively degrades excess single-stranded oligonucleotides while leaving double-stranded complexes intact. Probes hybridized to their target nucleic acid, or amplified products thereof or primers that are annealed to a probe thus remain present in the sample and can be identified in subsequent steps.

The hybridized single-stranded DNA oligonucleotide probes are preferably identified by sequencing. The herein disclosed method utilizes single-stranded DNA oligonucleotide probes capable of specifically hybridizing to target nucleic acids molecules. In contrast to FISH-based detection methods, probe detection and identification according to the method of the invention do not require, and preferably, do not use, imaging-based methods, e.g., microscopic methods. Rather, probes hybridized to a target nucleic acid are identified, e.g., via sequencing. Probes may be sequenced by any suitable method known in the art. Small sets of selected probes may for instance be sequenced by sequencing methods that rely on e.g., incorporation of chain-terminating dideoxynucleotides during in vitro DNA replication (e.g. Sanger-sequencing).

In a preferred embodiment, probes are identified by next generation sequencing (NGS). Due to the short size of the oligonucleotide probes of the invention, sequencing may be performed using commonly known short-read high-throughput NGS technologies as provided, e.g., by Illumina, Roche 454, Helicos, PacBio, SOLiD and Complete Genomics. Dependent on the precise NGS platform to be used, samples need to be prepared accordingly, i.e., oligonucleotide probes have to be appended to appropriate sequencing adaptors and samples may need to be loaded onto a suitable flow cell. Sequencing adaptors can be readily ligated to the end of each probe or added during PCR-based probe amplification to generate a sequencing library. Illumina for instance provides multiple kits for sequencing library preparation (e.g. Nextera Flex Library Prep Kit). It is particularly advantageous that the method of the invention circumvents the need for isolating RNA from the compartment and reverse transcribing it into cDNA for detection of RNA, since the method of the invention only determines the sequence of DNA oligonucleotides complementary to RNAs. Both RNA isolation and reverse transcription are known to harbor great risks of material loss and potentially introduce biases to the final analysis.

Sequencing provides a discrete count of probes per unique target site on the nucleic acid, e.g., RNA, species of interest. Obtained sequencing reads may subsequently be aligned to a probe reference map which contains all probe sequence information, including probe specific barcodes, primers and/or UMIs. Probes may next be ordered by their genomic position in a reference genome. Summary statistics may then be applied, for example the raw count of the number of probes detected across a whole transcript or exons/introns.

In a preferred embodiment, after step c) and, typically, after extraction of the single-stranded DNA oligonucleotides that have specifically hybridized to a nucleic acid within the compartment from the compartment, in step d), the single-stranded DNA-oligonucleotide probes specifically hybridized to nucleic acid molecules within said compartment are identified by probe amplification and probe sequencing, in that order.

In one embodiment, the entire length of a probe is sequenced. For instance, sequencing may initiate at either the 3' or the 5' end of the probe and subsequently continue to the opposing end. To reduce costs and increase the throughput of the method, probes may alternatively be sequenced only partially, i.e. starting from an identical starting position at either the 3' or 5' end, only the first 30, the first 35, the first 40, the first 45, the first 50, the first 55, the first 60, the first 65, the first 70, the first 75, the first 80, the first 85, the first 90, the first 95 or the first 100 nt of each probe may be sequenced. In an embodiment described in the examples, sequencing initiates at the 5' end, and each sequencing read has a length of 75 nt. Therefore, sequencing read lengths according to the method of the invention are considerably shorter than those used in conventional RNA-seq experiments (about 200 nt), thus saving monetary costs and time. In case probes contain flanking barcode sequences, sequencing read length may be further reduced to cover only a sufficient length of the flanking barcode sufficient to identify the target RNA. Incorporation of UMI sequences into the probe may furthermore facilitate the identification of amplification artifacts such as PCR-caused duplications and do not prevent identification of RNA species present in the sample.

In an alternative embodiment, paired-end sequencing may be performed, wherein each probe is simultaneously sequenced from both the 3' and the 5' end, to improve sequencing sensitivity and to detect duplicates that may have arisen as a consequence of technical PCR biases.

Advantageously, in the method of the invention, probe sequencing includes sequencing of at least a part, optionally all of the region of the probe that had hybridized to the target nucleic acid, i.e., has a sequence complementary to the target region. That avoids the need for a complicated probe design and allows for shorter probes.

Albeit becoming more affordable, sequencing costs still represent a limiting factor for many labs, especially when sample sizes are high or repeated analyses are required. In addition, interpretation of sequencing results requires the work of trained personnel and high computing power. Detection and quantification of selected and relatively small RNA subsets may be more cost-efficient and readily achieved by more conventional methods such as Northern blotting or reverse transcription quantitative PCR (RT-qPCR) that do not involve a sequencing step. However, both techniques require RNA to be isolated and further processed and thus harbor a considerable risk of losing precious material due to sample degradation. Northern blotting furthermore often lacks sensitivity to sufficiently detect lowly abundant transcripts. RT-qPCR by contrast relies on reverse transcription of the target RNA.

In an alternative embodiment, the method of the present invention also allows for identification of hybridized single-stranded DNA oligonucleotide probes by amplification, preferably by quantitative PCR, without the need for sequencing technologies (Fig. 5b, Fig. 6a, and Fig. 6b). Primers may be designed that specifically target the barcode sequences of a probe complementary to a specific gene of interest, and PCR, e.g., quantitative PCR, will result in their amplification. Different suitable protocols and reagents for qPCR analysis of nucleic acids are known in the state of the art. E.g., they are readily available from companies such as Sigma-Aldrich, Thermo-Fisher, Promega and others. Detecting oligonucleotide probes via PCR-based amplification thus allows for a quick way of monitoring the expression particularly of few selected genes. Again, advantageously, the region of the probe that has hybridized to the target nucleic acid is amplified.

The method of the invention further allows for spatially mapping of detected nucleic acids, e.g., RNA, in the compartment, further comprising the steps of
(i) sectioning, in particular cryosectioning or cryomilling, preferably, cryosectioning, the compartment prior to step (b) to obtain a collection of fractions and thus separating nucleic acid molecules from each other depending on their localization;
(ii) identifying the single-stranded DNA-oligonucleotide probes specifically hybridized to nucleic acid molecules within each fraction in step (d) and thus determining the presence or absence of nucleic acid corresponding to the probe in each fraction; and
(iii) mapping nucleic acid within the compartment.

In the context of the invention, spatial mapping is understood to mean that the spatial localization or position of a nucleic acid is to be determined within a compartment. Existing methods used to spatially map nucleic acids in a cell or subcellular region often rely on FISH-based imaging methods that are either low-throughput or require expensive equipment. Spatial transcriptomics so far relies on massive parallel sequencing of cDNAs derived from RNA captured on pre-designed probe arrays, but their use is limited to tissue sections.

By contrast, the method of the invention combines ultracryosectioning of an individual compartment (e.g., a single cell, cytoplasm or cell nucleus) with indirect detection of target nucleic acids by sequencing only those ssDNA oligonucleotide probes that hybridized to nucleic acids within said compartment. Other types of sectioning that do not involve freezing of the compartment may also be compatible with the invention. For instance, in one embodiment, the compartment may also be formalin-fixed paraffin-embedded (FFPE), i.e. first fixated in formaldehyde to preserve the structural integrity of the compartment prior to its embedding into a paraffin block than can subsequently be sliced into distinct sections.

When thin cryosections, as described herein, are cut through the compartment, probes against e.g. RNA molecules that are either transcribed, translated or fulfil e.g. a regulatory function in a common locality of that compartment are detected in the same section more often than probes against RNA molecules with functions in other areas. In case of cryosectioning, e.g., a cell nucleus, the site of origin of a nascent pre-mRNA transcript may thus be inferred by scoring the presence or absence of copies of said pre-mRNA (i.e. the abundance of probe oligos covering intronic regions or exon-intron and intron-exon junctions) among a number of sections through that individual nucleus. Similarly, when cryosectioning cytoplasm of a single cell, the method may thus infer the subcellular localization where, e.g., a regulatory non-coding RNA may preferentially exert its biological function or where an mRNA is translated into protein (e.g. by selecting the leading edge of a fibroblast, or neuronal axons or dendrites). The method of the invention may also provide information on the relative distance between individual nucleic acid, e.g., RNA, molecules within the compartment space. Thus, the results of this analysis may be used to e.g. compute the co-segregation frequency of each RNA molecule against every other RNA molecule detected by the method of the invention to create a matrix of inferred relative distances between RNA transcripts.

Therefore, in one embodiment of the invention, all sections of a compartment are analyzed by the method of the invention, rendering the spatial mapping of nucleic acids in a single cell possible. However, this is not required, and the analyzed fractions may be sampled from a plurality of compartments, e.g., a plurality of single cells or nuclei, across the population of cells of interest. Using the method of the invention, preferably, more than 180 fractions are analyzed for the presence or absence of probes targeting certain nucleic acids and, optionally, probe co-segregations. For example, about 180 to about 10000 fractions, preferably, about 200 to 5000, about 220 to 4000, about 230-3500, about 250-3000, 300-2000 or 500-1000 fractions may be analyzed, wherein these fractions may be obtained from a single nucleic acid-containing compartment or a plurality of nucleic acid-containing compartments.

Investigating spatial distribution of nucleic acids in a compartment may be complemented by analysis of nucleic acid co-segregation, which may be achieved with a statistical analysis to determine spatial proximity (e.g. Weibel, E.R., 1979, Stereological Methods: Practical Methods for Biological Morphometry. Vol. 1 Academic Press, London, UK; Weibel, E.R., 1980, Stereological Methods: Theoretical Foundations. Vol. 2. Academic Press, London, UK). This may be of particular interest as close spatial proximity between e.g. two RNA molecules with sequence similarities can be a sign for e.g. a common transcriptional origin of the two RNAs. The two RNA molecules may for instance be unknown splice variants or isoforms derived from a common gene. By contrast, close spatial proximity of two clearly distinct RNA molecules and/or species in a nuclear fraction may suggest that two independent genes are expressed at similar time points in close distance to each other, which could in turn suggest that both genes share common regulatory elements. Dependent on the context and the precise sequence of the two RNA molecules, a close proximity may also suggest regulatory interactions between them. Co-segregation of viral RNA molecules and small RNAs in a common cytoplasmic fraction may furthermore suggest e.g. an active RNAi response. Analyzing co-segregation of RNA molecules with a statistical method may thus not only contribute to spatial mapping of a plurality of RNA molecules but may also provide novel insights into transcriptional and post-transcriptional relationships between RNAs as well as antiviral host responses. In addition, co-detection of two or more RNA species in one single cell, group of cells or section of a tissue (e.g. sampling a tissue through sampling RNA content in an equally sized square, or other shape) can be used to reconstruct the cell type content and cell-to-cell spatial relationship in a complex tissue. Statistical methods used in the method of the invention may be, e.g., inferential statistic methods.

In one embodiment, the position of a nucleic acid is determined when a probe against said nucleic acid is detected at a frequency higher than a pre-set threshold to reduce the risk of mismapping nucleic acid molecules to areas due to e.g. fixation artifacts or RNA/DNA contaminations. The threshold may have to be adjusted dependent on the expected abundance of a nucleic acid in the compartment. The position of nucleic acids such as RNAs, especially very rare nucleic acid species whose detection frequency might fall below the expected threshold, may be verified when copies of said nucleic acids are detected repeatedly in the same or similar compartment fractions in different biological replicates. The method also has the potential to differentiate between nucleic acid, e.g., RNA-RNA interactions that occur randomly and those that may have a physiological function by integrating co-segregation data between two nucleic acids, e.g., RNAs, and data on sequence complementarity.

Determining the presence or absence of RNA may also comprise determining the quantity of the RNA.

Information on nucleic acid, e.g., RNA, localization may be studied, e.g., in the context of disease or upon environmental changes. For instance, the spatial distribution and abundance of RNA may be studied in cells of a healthy donor compared to corresponding cells of a donor with a medical condition, e.g. a human cancer patient. Comparisons may also be done between different groups of cells within a tissue, e.g. all neurons versus glia cells in a brain tissue, or tumor cells versus healthy cells in a tissue biopsy.

For analysis of the results of spatial mapping in the context of the invention, methods described in WO 2016156469 or Beagrie et al., 2017 Nature 543(7646), 519-524 may be used in a corresponding manner, e.g., statistical methods described therein.

Spatial mapping of nucleic acid molecules such as RNAs or ssDNAs may be combined with detection of additional biological molecules inside a compartment to gain novel insights on the interplay of e.g. gene expression with other vital processes in a cell.

Therefore, the method of the invention further allows for detection and spatial mapping of nucleic acids that preferably are RNA, in combination with the detection of at least one DNA locus within a compartment, comprising additional steps of
- determining the presence or absence of at least one DNA locus in each fraction, optionally, by sequencing, preferably by next generation sequencing; and
- determining co-segregation of said at least one DNA locus and the single-stranded DNA oligonucleotide probe(s) specifically hybridized to a nucleic acid, preferably an RNA.

A locus is the specific location of a gene, DNA sequence, or position on a chromosome. The locus may thus be the location of e.g. a protein-coding gene, a gene that is transcribed into an RNA molecule that does not encode a protein, a pseudogene, an enhancer region, a transposable element, a repetitive sequence or any DNA sequence of unknown function or no function at all. In a preferred embodiment, the at least one DNA locus is a genomic DNA locus. Genomic DNA in e.g. a eukaryotic cell nucleus is organized as chromatin, a compacted and dense structure consisting of DNA being wrapped around protein complexes formed by histones.

As RNA molecules are derived from transcribed genes, therefore, in one embodiment, detection and spatial mapping of RNAs in a compartment may be combined with the detection of a gene of interest or a particular part thereof. This can be achieved, e.g., by parallelly isolating and sequencing both genomic DNA and ssDNA oligonucleotide probes bound to RNAs from the same nuclear fractions to identify those loci (e.g., genes) that co-segregate with the probes. In another embodiment, detected probes may not be analyzed for co-segregation with a gene or a particular region thereof, but instead for co-segregation with a regulatory DNA locus such as an enhancer region. Co-segregations of genomic DNA loci and DNAs of interest (e.g. viral ssDNAs or ssDNA in R-loops) may be determined analogously.

Preferably, nucleic acid, e.g., RNA or ssDNA, detection and spatial mapping is combined with detection of more than one genomic DNA locus, e.g., 2, 5, 10, 50, 100, 500, 1000, or more genomic DNA loci. In a particular embodiment, RNA detection and spatial mapping is combined with the analysis of the entire genome.

One of the main interests pursued by the inventors is to understand the interplay between the regulation of gene expression and genome architecture. Chromatin exists in interacting and non-interacting states. Studying the structural properties and spatial organization of chromatin is important for the understanding and evaluation of the regulation of gene expression. A method known as optical reconstruction of chromatin architecture (ORCA) developed by Mateo et al., 2019, (Visualizing DNA folding and RNA in embryos at single-cell resolution. Nature 568, 49-54) relies on oligopaint probes to three-dimensionally reconstruct genomic organization of regions of about 100-700 kb. In that study, ORCA was combined with single-molecule RNA-FISH to examine the expression of 30 RNA species and relate it to local chromatin organization. Therefore, existing methods like ORCA are limited to capturing information from relatively short genomic regions and detection of only small subsets of RNAs, or relying in extensive number of steps of sequential hybridization and imaging.

The inventors overcame these limitations by combining high-throughput RNA detection according to the method of the invention with a recently developed and innovative sequencing-based method for analyzing the three-dimensional structure of DNA in a whole compartment, e.g. a single cell, nucleus or organelle. Said method has been termed genome architecture mapping (GAM) by the inventors. A detailed account on the method of GAM is provided by Beagrie et al., 2017 Nature 543(7646), 519-524 and WO 2016156469. In brief, GAM uses next generation sequencing of genomic DNA and statistical methods to compute the spatial proximity of multiple DNA loci by determining their co-segregation amongst fractions of a compartment, preferably a cell nucleus. In consequence, GAM allows for in-depth analysis of higher-order chromatin interactions, including the identification of binding sites genome wide in an unbiased manner and thus provides a detailed map of genomic architecture. GAM also allows whole genome haplo-type reconstruction (Markowski et al., 2020, GAMIBHEAR: whole-genome haplotype reconstruction from Genome Architecture Mapping data. bioRxiv 2020.01.30.927061), which combined with the invention can enable the study of allele-specific gene expression, i.e., the mechanisms by which genetic variants regulate gene expression.

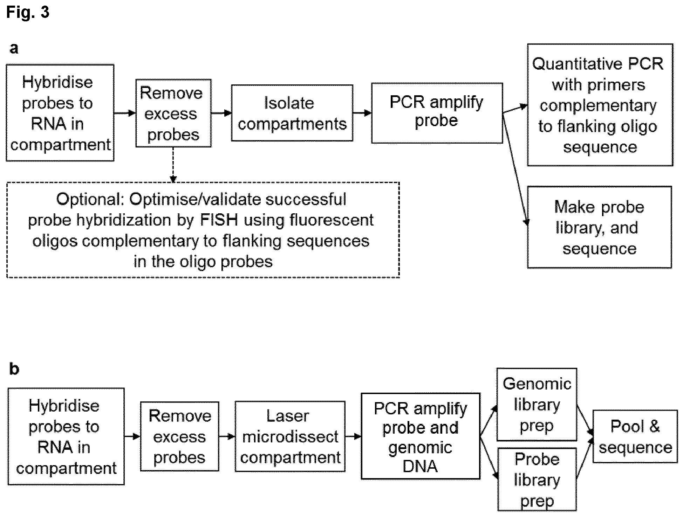

To combine RNA detection according to the method of the invention with GAM analysis, a compartment, e.g. a cell nucleus, may preferably be sectioned into ultrathin fractions, e.g. via cryosectioning. Single nuclear profiles (NPs) (or profiles of other nucleic acid containing compartments, e.g., mitochondria) are isolated from these fractions, e.g., by laser microdissection. From each profile, both genomic DNA as well as RNA-bound DNA oligonucleotide probes that were retained in the compartment after stringent washing are sequentially or, preferably, simultaneously, isolated and PCR-amplified. Probe and genomic DNA may next be indexed separately to generate two distinct sequencing libraries (one for the oligonucleotide probes and one for the genomic DNA). The two libraries may then be pooled together and sequenced (Fig. 3b). Every sample that is to be tested by the method of the invention may thus produce two independent sequencing files. Read recovery for oligonucleotide probes may be quantified as a proxy for relative abundance of RNA species in the original RNA-containing compartment and may be used to cluster individual compartment fractions before deconvolving cell-type/state specific three-dimensional genome topologies from genomic reads. Co-segregation of detected RNA molecules and genomic loci in each fraction may be determined. Parallel detection of RNA and genomic DNA from individual NPs thus enables the study of the interplay between chromatin topology and regulation of gene expression. Variations of the above described work-flow may be possible. For instance, the compartment may be any compartment that comprises both DNA and RNA, e.g. a mitochondrion or chloroplast or a prokaryotic cell. Fractions may also be derived from a plurality of compartments and co-segregation of RNA molecules and DNA loci may be compared for each of the corresponding fractions.

Preferably, co-segregation frequencies of multiple genomic DNA loci in distinct compartmental, e.g., nuclear, fractions may be analyzed to determine specific chromatin interactions, relative and absolute distances between loci and the radial positioning of loci within the nucleus. In consequence, the information may be used to infer chromatin architecture and topology in the compartment, e.g., the nucleus, and determine e.g. proximities between gene promoters and distant enhancer regions. At the same time, relative distances between DNA loci and detected RNA molecules may be inferred by scoring their presence or absence among a number of sections through individual nuclei. As a result, the co-segregation frequency of each detected RNA molecule against any DNA locus may be computed to create a matrix of inferred relative distances between RNAs and genomic loci. The statistical methods used to analyze co-segregation of RNA molecules and DNA loci correspond to the methods used to analyze co-segregation of DNA loci during GAM analysis or of distinct RNA molecules as describe above.

Information on gene expression in relation to genome architecture may be exploited in several different ways:
In one embodiment, genome architecture may be directly related to spatial mapping of expressed RNAs to determine the effect of chromatin topology and architecture on transcription, RNA processing and mRNA expression. For example, in case GAM analysis provides evidence for specific promoter-enhancer associations, the present invention may demonstrate whether or not these associations coincide in increased transcriptional output. Previous studies using ORCA surprisingly suggested that promoter proximity to enhancers was present, yet weak, at sites of active genes characterized by high levels of nascent RNA (Mateo et al., 2019). This study was however limited to relatively short genomic regions and few genes, in embryos of the model system Drosophila melanogaster. The present invention has the potential to expand such studies to the entire genome and in mammalian or plant cells.

In another embodiment, the method may also demonstrate how changes in gene expression may affect chromatin topology. For instance, detection of a nascent eRNA expressed from an enhancer region may be tested for the eRNA's ability to induce alterations to chromatin organization, e.g., to bring its corresponding enhancer into the vicinity of a target promoter.

In yet another embodiment, information on gene expression may be used as a read-out for cell state changes and their effect on genomic structure and gene position. A well-researched example for cell state is the cell cycle. Different cell cycle stages are associated with drastic changes in overall genomic structure (Nagano, T., 2017, Cell-cycle Dynamics of Chromosomal Organization at Single-Cell Resolution. Nature, 547(7661):61-67). By targeting RNAs of key cell cycle marker genes, laser microdissected nuclear slices from an intact tissue can be conveniently categorized based on their time point through the cell cycle, thus allowing an improved pinpointing of cells at specific stages of the cell cycle, while retaining spatial resolution. Similarly, RNA detection with the method of the invention may facilitate the identification of specific cell types or stem cell differentiation states to enable the study of characteristic chromatin rearrangements associated therewith.

Dependent on the degree of DNA compaction around histones, chromatin may be organized into more or less condensed higher-order structures. In its less condensed form, DNA is wrapped around histone proteins to form nucleosomes that can be evenly spaced from each other and thus resemble an unfolded set of beads on a string. Such loosely packed chromatin is referred to as euchromatin. Euchromatin is associated with active transcription of genes into RNA since genes are more readily accessible to the transcription machinery and auxiliary transcription factors. By contrast, heterochromatin refers to highly condensed, tightly packed chromatin structures where individual nucleosomes wrap into thicker higher-order chromatin fibers. Due to its high degree of compaction, heterochromatin is not well accessed by the transcription machinery and thus remains transcriptionally silent. Although both centromeres and telomeres are usually in a constant heterochromatic state, chromatin condensation in other chromosome parts is a highly dynamic process that is controlled via post-translational modifications of histone proteins that can be epigenetically inherited. GAM is also able to provide information on chromatin condensation states at the scale of tens of kilo- or megabases as well as whole chromosomes (Beagrie et al., 2017). Therefore, by combining the method of the present invention, designated oligo-Seq with GAM, it may further be possible to link RNA expression to chromatin condensation and remodeling at a larger scale (approximately 300 kb or higher).

The skilled person may conceive of numerous additional applications and uses for a combined GAM and oligo-seq analysis. Any such applications and uses are herewith encompassed by the scope of the invention.

**Tab. 1** summarizes unique advantages of a combined oligo-seq and GAM analysis.

**Tab. 1 Advantages of a combination of oligo-seq with GAM**

| **Advantages of oligo-seq** | **Opportunities** |
|---|---|
| ***parallel measurements from the same cell:*** | Direct inference about relationships between genotype, 3D genome topology and cell state |
| - genotype and parental phasing | |
| - 3D genome topology | *(incl. molecular phenotypes, morphology and*/*or tissue position)* |
| - mRNA levels | |
| - other RNAs (e.g. introns) | |
| - morphology of cell/nucleus | |
| - position in tissue | |
| ***ligation-free 3D nucleome approach:*** | Detection of communities of 3D genome contacts, and relation to cell phenotype, to dissect higher-order nucleome |
| - quantitative access to multi-way 3D genome topologies | |
| ***physical slicing of structurally preserved nuclei captures:*** | Mapping genome association to the nuclear periphery and its relationship with molecular cell phenotypes, morphology and/or tissue position |
| - chromatin contacts spanning tens of megabases (e.g. between SEs) | |
| - radial position of genomic content - compaction of genomic regions | |

It is possible to combine most or all possible applications of a combined GAM and oligo-seq evaluation into a single multi-omics experiment to maximize the amount of information obtainable through the method of the invention.

The herein disclosed method for detection of nucleic acids such as RNA may not only be combined with the detection of genomic DNA loci. In another embodiment, RNA/DNA detection and spatial mapping may also be combined with the detection of DNA from an exogenous source, e.g. DNA from a virus. Viral DNA may be single- or double-stranded, and may be present in the cytosol of an infected cell or integrated into the host cell's genome. It may be present in some cells of a tissue and not others, or in the same cell type but with altered cell physiology (cell state). Co-segregation of RNA molecules and viral DNA in a fraction of a compartment may provide information on the extent, localization and consequences of viral proliferation and transcription, in intracellular, cellular or tissue-level cellular interactions.

In yet another embodiment, the at least one DNA locus that may be detected in combination with RNA may be a DNA oligonucleotide probe that labels a biological compound other than a nucleic acid present in the compartment. For instance, said DNA oligonucleotide probe may be attached to an antibody that specifically recognizes a protein of interest, or to a ssDNA tail in a guide RNA used with dead Cas9 labelling of a locus or a plurality of loci.

Accordingly, the method of the invention may also be used to combine detection and spatial mapping of RNAs with the detection of at least one protein, comprising
- contacting the compartment with a ligand, e.g., an antibody, capable of specifically binding to the protein that is labeled with an oligonucleotide probe, and
- determining co-segregation of said at least one oligonucleotide probe labeling the ligand, e.g., the antibody, and the single-stranded DNA oligonucleotide probe(s) specifically hybridized to a nucleic acid such as RNA.

The at least one protein detected in combination with nucleic acid, such as RNA, may be e.g. a protein that is directly encoded by an RNA detected via the method of the invention. It may also be a protein whose biosynthesis is suspected or known to be regulated by a detected RNA. The protein may further be suspected or known to interact with a given nucleic acid detected by the method of the invention. Accordingly, it may be a member of a protein-RNA or a protein-DNA complex. The protein may however also be a protein previously not associated with a particular nucleic acid. The protein may be endogenously expressed within the compartment or may be introduced from an exogenous source, e.g. via transfection. It may also be the result of viral gene expression. Optionally, more than one protein is detected in combination with nucleic acid in a compartment. For example, at least 2, at least 5, at least 10, at least 25, at least 50 or at least 100 proteins may be detected in combination with nucleic acid. Detection of a plurality of proteins can be used to assess cell physiology/state, and help relate a specific chromatin conformation (enhancer-promoter contact) with transcription and protein expression or post-translational modification.
The ligand-probe-conjugate, e.g., the antibody-probe-conjugate may be brought into contact with the compartment after fixation or vitrification. Preferably, contact is with a fraction of a compartment after sectioning, e.g., cryosectioning. It may however also be brought in contact with the compartment prior to sectioning, e.g. by detection of a genetically encoded probe, or frankenbody, to detect nascent or mature proteins (Zhao, N., et al. 2019, A Genetically Encoded Probe for Imaging Nascent and Mature HA-tagged Proteins in Vivo. Nat. Commun. 10(10: 2947.)

Parallel detection of ssDNA oligonucleotide probes hybridized to complementary nucleic acid molecules and DNA probes labeling ligands such as antibodies bound to proteins may e.g. be achieved in analogous fashion compared to the combined detection of RNA and DNA loci described above. In brief, after stringency washing, ssDNA oligonucleotide probes that had hybridized to complementary nucleic acids may be isolated from fractions of a compartment, e.g., cryosections, e.g. from a whole eukaryotic cell, a nucleus or the cytoplasm. In parallel, DNA oligonucleotide probes conjugated to ligands such as antibodies bound to proteins are released from the ligand, e.g., with a suitable chemical solvent (e.g., a salt buffer containing dithiothreitol (DTT)) and are isolated from the respective fractions as well. Both DNA oligonucleotide probe collections thus obtained may optionally be PCR-amplified and separately indexed to generate two distinct sequencing libraries (one for detected nucleic acids and one for detected proteins). The two libraries may then be pooled together and sequenced. Every sample that is to be tested by the method of the invention thus produces two independent sequencing files. By assessing co-segregation of probes detecting nucleic acid molecules and probes detecting proteins in a fraction, physical proximities between nucleic acids and proteins may be inferred. Combining detection of e.g. RNA and proteins as well as determining their spatial distribution in general and relative to each other within the compartment by the method of the invention may thus e.g. allow conclusions about the rate and level of RNA translation into protein. Close proximities between particular nucleic acids and proteins may further suggest possible interactions or the formation of biologically relevant RNA/DNA-protein-complexes.

In another embodiment, parallel detection and mapping of RNA and at least one protein may further be combined with the detection and mapping of at least one DNA locus, e.g. via GAM. This approach has the potential to link chromatin organization to both regulation of transcription and translation.

The level of gene expression also depends on the local accessibility of chromatin to the transcriptional machinery. Chromatin accessibility relates to the extent to which e.g. transcription factors may bind to a DNA locus to promote or repress gene transcription. Although GAM analyses on genomic structure allow conclusions to be drawn about the larger scale condensation and structural organization of chromatin, other methods such as ATAC-Seq are more specialized in determining the accessibility of chromatin at the level of single nucleosomes for the transcriptional machinery across the genome (Buenrostro, J. D., Wu, B., Chang, H. Y., & Greenleaf, W. J., 2015, ATAC-seq: A Method for Assaying Chromatin Accessibility Genome-Wide. Current protocols in molecular biology, 109, 21.29.1-21.29.9). ATAC-Seq thus represents a useful tool to map transcription factor binding sites.

Detection of nucleic acids, preferably RNA, according to the invention can also be combined with an analysis of local chromatin accessibility within the compartment, comprising the steps of
(i) cryosectioning or cryomilling, preferably, cryosectioning, the compartment prior to step (b) to obtain a collection of fractions and thus separating nucleic acid molecules from each other depending on their localization;
(ii) isolating genomic DNA from each fraction
(iii) simultaneously fragmenting and tagmenting the genomic DNA isolated from each fraction to generate an ATAC-Seq library;
(iv) purifying and, optionally, amplifying the ATAC-Seq library;
(v) determining the state of local chromatin accessibility for any given locus of the genomic DNA; and
(vi) analyzing the presence and/or abundance of mapped nucleic acid, preferably RNA and relating it to the chromatin accessibility at any given locus of the genomic DNA.

Assay for Transposase-Accessible Chromatin with high-throughput sequencing (ATAC-Seq) is a NGS-based method suitable for assessing the regulatory landscape of chromatin in a cell nucleus. ATAC-Seq relies on the activity of a hyperactive Tn5 transposase. Transposases naturally catalyze the movement of transposable elements from one part of the genome to another by a cut-and-paste mechanism. During ATAC-Seq, NGS-adaptors are loaded onto the Tn5 transposase. The loaded transposase next cleaves DNA at accessible, euchromatic chromatin regions (fragmentation) while simultaneously attaching the NGS-adaptors to these chromatin fragments (tagmentation) to generate an ATAC-Seq sequencing library. The library is purified and may be PCR-amplified using barcoded primers before being analyzed by qPCR or NGS. ATAC-Seq is usually performed with about 25,000-75,000 cells.

Detection of local chromatin accessibility via ATAC-Seq may be combined with nucleic acid, preferably RNA detection by the method of the invention. For instance, genomic DNA isolated from a cell or cell section may first be subjected ATAC-Seq to determine the chromatin accessibility state of different DNA loci in a nucleus. Changes in chromatin accessibility may then be tested for their effect on local transcriptional activity by assessing the presence of absence of previously mapped RNAs in the vicinity of a given locus.

In a preferred embodiment, the ATAC-Seq sequencing library and the library of hybridized ssDNA oligonucleotide probes are generated from the same compartment and are sequenced in a single sequencing run.

By assessing cells at distinct states or time points after a treatment, the method may also e.g. help to determine whether increased levels of RNA at a genomic locus are a consequence of increased transcription due to changes in local chromatin accessibility or, vice versa, expressed RNA molecules influence chromatin accessibility as previously suggested in C. *elegans* (Fields et al., 2019, Chromatin Compaction by Small RNAs and the Nuclear RNAi Machinery in C. elegans. Scientific reports 9). In consequence, the method of the invention allows for deciphering the dynamics of chromatin accessibility and gene expression.

The present invention also provides the use of the method of the invention for
(a) determining gene expression in single cells, groups of cells or intracellular compartments;
(b) identifying isoforms and allele-specific variants of RNAs within a compartment;
(c) quantifying transcription of genes;
(d) identifying cell types and states of complex heterogenous tissue;
(e) identifying endogenous and exogenous dsDNA and ssDNA within a compartment;
(f) mapping nucleic acid, e.g., RNA, location in the compartment;
(g) mapping RNA and nucleic acid loci location in the compartment;
(h) mapping nucleic acid, preferably RNA and protein location in the compartment; and/or
(i) mapping nucleic acid, preferably RNA, protein and nucleic acid loci location in the compartment.

For example, gene expression can be determined in a single cell in the context of the surrounding tissue, or in a group of cells in the context of the surrounding tissue, optionally, in cells of at least one, e.g., several, cell types of a specific tissue. Analysis may then involve comparison of different cells, e.g., different cell types. The invention thus allows for spatial transcriptomics of certain cells within the 2D or even 3D context of a tissue. Mapping can also be at the tissue/organ level and even populations of different individuals of different species, e.g., different single cell organisms or different prokaryotes.

The present invention further provides a method of diagnosing a disease associated with misexpression of one or more genes and/or a distinct transcriptional profile in a patient, comprising, in a sample taken from said patient, identifying the presence and/or analyzing the abundance of nucleic acids, in particular RNAs in the patient to obtain a patient-specific transcriptional profile, and comparing said patient-specific transcriptional profile with the transcriptional profile of a subject already diagnosed with said disease, wherein the transcriptional profile is preferably also compared with the transcriptional profile in a healthy subject. Alternatively, transcriptional profiles may be compared between specific subgroups of cells, which may be derived from the same patient, e.g., tumor cells and normal tissue, preferably, normal tissue from the same cell type as the tumor tissue.

As the present invention may be used to investigate disturbed gene expression in a patient, e.g., over-expression of oncogenes associated with tumorigenic cell growth, it may also contribute to treatment of patients having a disease associated with disturbed gene expression, e.g., cancer.

In summary, the invention provides a highly sensitive sequencing or amplification-based method for detecting nucleic acids in a sample. In contrast to known RNA sequencing methods, the method of the present invention does not rely on either RNA isolation or cDNA generation as prerequisite for RNA detection. It thus overcomes problems associated with rapid RNA degradation or biases introduced during reverse transcription. Instead, the herein introduced method relies on sequencing ssDNA oligonucleotide probes that have complementarily hybridized to nucleic acids inside a tissue, cell or organelle.

Table 2 summarizes differences between the method of the invention, designated oligo-seq, and state of the art methods.

**Tab. 2. Table listing features of oligo-seq in comparison with state-of-the-art methods**

| **Critical steps** | **scRNA-seq** | **Spatial transcriptomics** | **MerFISH I ORCA** | **HyPR-seq** | **oligo-seq** | **Preferred option** |
|---|---|---|---|---|---|---|
| 1. Requirement for cell dissociation prior to RNA detection (compatibility with cell dissociation) | | | | | | |
| | Y | N | N (not tested?) | N | N (Y) | N (to avoid perturbation of physiology, loss of RNA) |

| 2. RNA extraction from cell | | | | | | |
|---|---|---|---|---|---|---|
| | Y | Y | N | N | N | N |
| | | | | | | (to avoid RNA degradation) |

| 2. Preservation of RNA integrity and content | | | | | | |
|---|---|---|---|---|---|---|
| | No fixation, RNAs are extracte d | No fixation or methanol fixation, RNAs are extracted | Weak fixation, RNAs are not extracted | Weak fixation, RNAs are not extracte d | Weak, strong or methano I fixation, RNAs are not extracte d | Good extraction or good preservation |

| 3. RNA detection based on reverse transcription of RNA | | | | | | |
|---|---|---|---|---|---|---|
| | Y | Y | N | N | N | N (for lower costs, robustness to partial degradation) |

| 4. RNA detection based on hybridization of oligos | | | | | | |
|---|---|---|---|---|---|---|
| | N | N | Y | Y | Y | - |

| 5. Number of sequential hybridizations required for signal amplification or enhanced sensitivity (minimal length of longest oligo required) | | | | | | |
|---|---|---|---|---|---|---|
| | - | - | 16/>2 (∼120bp or longer) | 3 (75 bp) | 1 (-75 bp) | Lowest (shortest) |

| 6. Detection of oligos based on fluorescence | | | | | | |
|---|---|---|---|---|---|---|
| | - | - | Y | N | Y/N | - |

| 7. Detection of oligos based on ligation between hybridized oligos | | | | | | |
|---|---|---|---|---|---|---|
| | - | - | N | Y | N | N (for lower costs) |

In their recent study, Marshall et al. proposed a similar concept when they developed HyPR-Seq. However, the present invention provides a series of decisive advantages over HyPR-Seq: Firstly, the specificity of the present method is based foremost on the length and number of the used probes as well as the chosen incubation times, temperatures and stringency washes. HyPR-Seq utilizes two initial probes of 25 nt length. The target recognition site of the probes used in the method of the invention preferably are at least about 32 nt in length, thus exhibiting a higher specificity. Secondly, HyPR-Seq is a far more time-consuming and laborious method, as it involves 8 distinct consecutive hybridization and washing steps prior to amplification and sequencing. By comparison, oligo-seq only involves a single hybridization step followed by stringent washing. As admitted by the developers of HyPR-Seq, multiple rounds of hybridization and washing may lead to significant cell loss. Therefore HyPR-Seq requires starting an experiment with at least 1 million cells. The method of the invention, in contrast, may be successfully done with a single compartment of 1/20^{th} of a mammalian cell. In addition, the numerous washing steps of the HyPR-Seq protocol and a harsh ethanol permeabilization may affect the structural integrity and protein content of the nucleic acid-containing compartment. Therefore, HyPR-Seq may not be as compatible with other multi-omics techniques. The method of the invention furthermore requires only a single layer of oligonucleotide hybridization, and thus does not rely on an amplification of the primary RNA-DNA hybridization event by hybridization of additional secondary or tertiary oligonucleotides, as compared to HyPR-Seq. Accordingly, oligo-seq enables parallel identification of a significantly higher number of nucleic acids as HyPR-Seq or, for that matter, most other FISH probe-based detection methods.

Table 3 again summarizes the differences between the method of the invention and HyPR-Seq.

**Tab. 3 Comparison between HyPR-Seq and oligo-seq**

| **POINT** | **HYPR-SEQ** | **OLIGO-SEQ** | **NOTES** |
|---|---|---|---|
| Specificity | Achieved by ensuring consecutively bound Primary probes | Achieved by the length of oligos and number of oligos targeting each transcript, and by management of thermodynamic properties and stringent washes, i.e. hybridization/stringen t wash temperature and length of respective incubations | remaining primary probe after stringent washes in both cases will lead to reduced specificity in sequencing data. If HyPR-Seq were to increase the specificity with stringent washes by increasing target site length, this would need to be reflected in both targeting probes hence greatly increasing the required target site |
| Length of required target site (as of current data) | 2x25nts + 2nt spacer | -35 nts | This difference in length of target sites makes detecting some RNA species unachievable for HyPR-Seq due to length. |
| | | | Also increasing target length reduces the number of target sites possible per qene |
| Number of incubations and washes required, before amplification and sequencing | 8 steps: | 2 steps: | Increased wash steps may incur the unwanted removal of RNA |
| | 1) primary probe | 1) primary probe | |
| | 2) wash | 2) wash | |
| | 3) H1 Hairpin | | |
| | 4) wash | | |
| | 5) read out probe | | |
| | 6) wash | | |
| | 7) ligation | | |
| | 8) wash | | |
| Compatibility with other omics techniques | Compatibility with multiomics techniques that require preservation of protein content and nuclear structure may not be as efficient with the harsh ethanol permeabilization required and the increased wash and mechanical stress taken place due to increased wash steps. | Successfully applied in formaldehyde-crosslinked which preserve the content of proteins and their post-translational modifications, RNA and DNA (cf. Guillot 2004, Branco & Pombo 2006, Ferrai et al. 2010). | Examples of single cell omics techniques include scHi-C, GAM, scATAC, Cut&Run, Cut&Tag, CITE-Seq, ChILT |
| Accessibility to target site | Requires access: 1) ssDNA oligos with 74 nt length (hairpin oligo B1H1) 2) T4 Liqase | Requires access of ssDNA oligos with <75 nt length | |

The method of the invention effectively and faithfully provides information on e.g. gene expression, even when dealing with very low amounts of starting RNA material. Therefore, it is particularly suitable for studying gene expression at the single cell, or even subcellular level. In combination with tissue or cell sectioning, the method according to the invention further allows to monitor spatial distribution of nucleic acids in relation to other biomolecules within a compartment, thus providing a highly sensitive technique suitable for conduction spatial transcriptomics. Detection of nucleic acids according to the invention does not rely on any microscopic or imaging technologies, but rather uses sequencing to provide transcriptomic data. It thus can be readily combined with other sequencing-based methods known in the art in larger multi-omics studies.

Throughout the invention, the term "about" is intended to be understood as "+/- 10%". If "about" relates to a range, it refers to both lower and upper limit of the range. "A" is intended to mean "one or more", if not explicitly mentioned otherwise.

All literature cited herein is herewith fully incorporated. The present invention is further illustrated, but not limited, by the following examples.

### Brief description of the Drawings

**Fig. 1** Schematic of probe designs comprising the design used in the below experiment (design A) and oligo designs B and C. The Universal Primer (UP) is 20 nucleotides and shared by all probes in the library. UP1, UP2 and UP3 denote different universal primer sequences. Unique Molecular Identifiers (UMI) are stretches of 6 to 15 random nucleotides that identify unique molecules in sequencing, and if found repeated due to PCR amplification are counted only once. The homology region (HR) is the sequence that targets a unique nucleic sequence of the target molecule of interest. Each probe in the library has a unique homology region. The 5' barcode B1 used in the example is specific to all probes targeting the same gene, the 3' Barcode B2 is specific to either all exons or introns targeting the same gene.

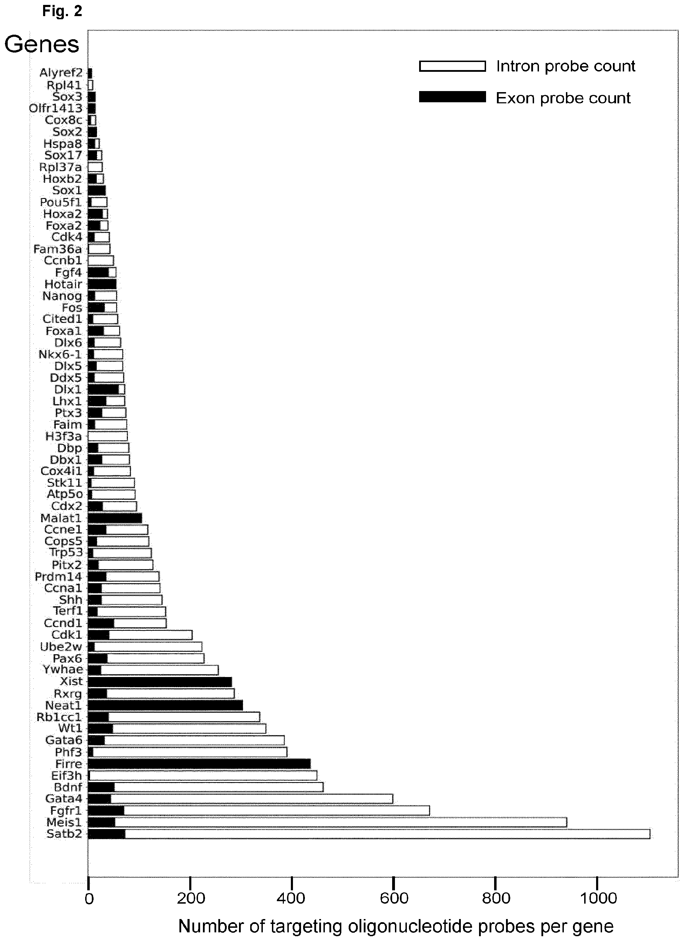

**Fig. 2** List of 66 genes included in probe design A, and number of targeting oligos per gene, in exons or introns. Hotair, Malat1, Xist, Neat1 and Firre encode long-noncoding RNAs, and other genes encode for messenger RNAs.
**Fig. 3** Schematics of the method of the present invention. (**a**) RNA detection in compartment. Optional validation of specific probe hybridization by fluorescence in situ hybridization (FISH) is shown in dotted box. (**b**) RNA detection in compartment combined with parallel DNA detection using GAM procedures.
**Fig. 4** Validation of probe library hybridization using RNA-FISH and of hybridization specificity using RNAse treatment prior to hybridization. **(a)** Top panels show the hybridization of ultrathin (200 nm-thick) cryosections of mESCs (clone F123) with the designed RNA-GAM probe (concentration 0.5 µM). The bottom panels show a hybridization performed in parallel in cryosections pre-treated with 0.25 mg/ml RNAse A for 2 h. Left panels represent DNA staining with DAPI, to identify nuclear slices. Right panels show the probe signal detected after secondary hybridizations of the cryosections with oligos targeting the universal sequence of the ssDNA probes (bridge) followed by annealing of AlexaFluor647-conjugated oligos which hybridize to the bridge. Scale bar, 10 µm. **(b)** Schematic of fluorescence-based detection of hybridized oligo-seq primary probes.
**Fig. 5 (a)** Simplified overview of PCR steps used to sequence ssDNA probes used in the example. Probe design is the same as in Fig. 2. Illumina adapters P5 and P7 are complementary to the universal primer regions at the end of each probe (UP1 and UP2, respectively). **(b)** Schematic overview of qPCR steps used to amplify specific subsets of ssDNA probes, such as only those probes specific to a single gene or a single region within a specific gene. Probe design is the same as in Fig. 2. Probes are extracted from compartment of interest. Probes are then universally amplified with primers UP1 and UP2, and different samples are normalised by DNA concentration prior to qPCR. Relative abundance can then be determined for all probes targeting the same gene by amplifying the region between the 5' B1 barcode and the 3' UP2 with primers B1 and UP2. Alternatively, gene region regions may be specifically amplified by amplifying the probe region between B1 and B2 with primers B1 and B2.
**Fig. 6 (a)** qPCR results of oligo probe amplification from 100NP samples of mESCs-F123 (+), XEN cells (●) and RNAse-treated mESCs-F123 (▲). The Y axis represents the number of quantification cycles (Cq) required for detection of SYBR-Green signal. Expression of six genes was performed in independent replicate samples (each with 100NPs). Sox2 and Oct4 genes are pluripotency genes expressed specifically in mESCs-F123, Sox17 and Gata6 genes are expressed specifically in XEN cells, Malat1 is a long non-coding RNA which is abundantly and ubiquitously expressed in both mESC and XEN, and BDNF gene is a neuronal gene not expressed in mESCs or XEN. -1, and -2 denotes technical replicates 1 and 2. **(b)** qPCR results of oligo probe hybridization in whole cells in solution. mESCs (F123) were trypsinized, hybridized with oligo probes, and washed. The bound probe content of 1-20 cells was extracted and amplified (+). Amplified probes were cleaned and normalised to 2.5ng/µl. As background control, the probe stock was normalised to 2.5ng/µl (●). The Y axis represents the number of quantification cycles (Cq) required for detection of SYBR-Green signal. Sox2 and Oct4 genes are pluripotency genes expressed specifically in mESCs-F123, Sox17 and Gata6 genes are expressed specifically in XEN cells, Malat1 is a long non-coding RNA which is abundantly and ubiquitously expressed in both mESC and XEN, and BDNF gene is a neuronal gene not expressed in mESCs or XEN. Specific detection above probe background is found for mESC-expressed genes, Sox2, Oct4, and ubiquitously expressed Malat1. -1, and -2 denote technical replicates 1 and 2, respectively.

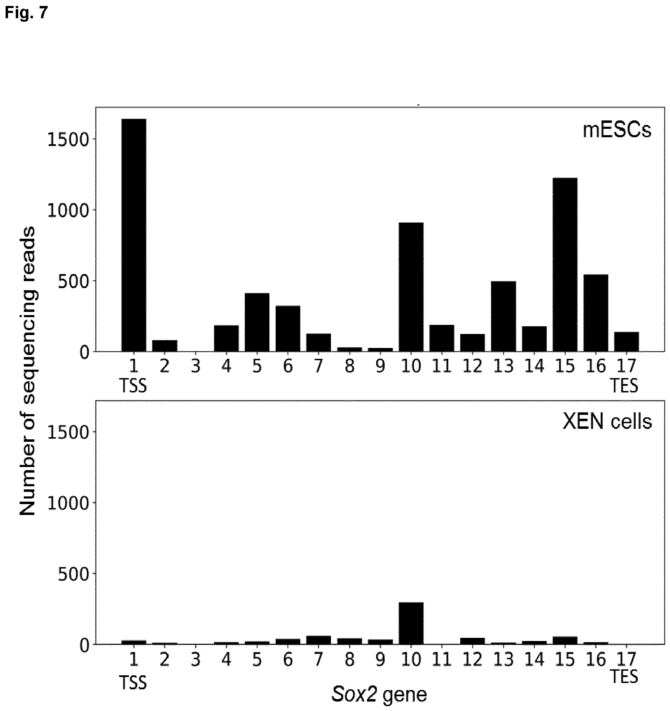

**Fig. 7****.** Number of hybridized probes in each homologous target site across the Sox2 gene in 100NP samples from mESCs or XEN cells. Sox2 is expressed in mESCs and not XEN cells. The probe library (design A) contains only a total of 17 oligonucleotides that target the Sox2 gene across its coding region, here ordered from left to right in genomic order (positions 1-17) from transcription start site (TSS) to transcription end site (TES). Y-axes represent the number of probes targeting that unique target site.

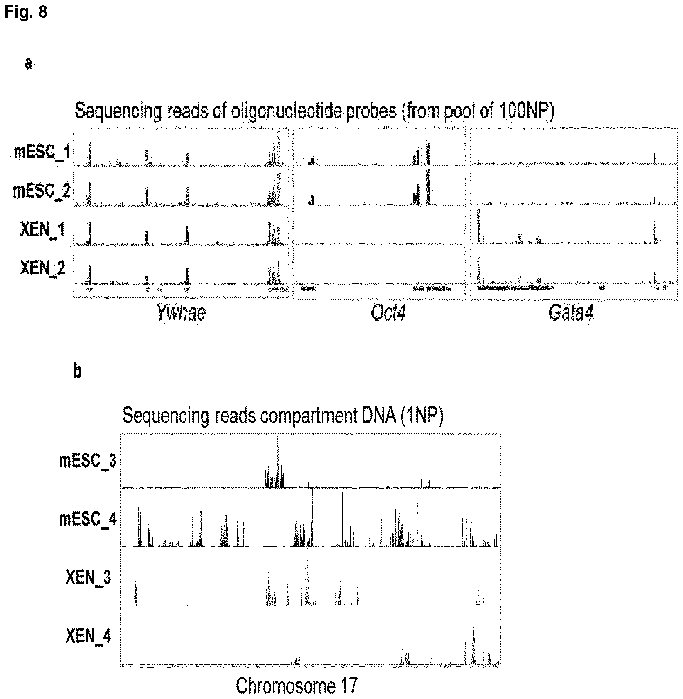

**Fig. 8****.** Parallel detection of RNA abundance and DNA presence in RNA-GAM. Ultrathin cryosections from mESCs and XEN cells were hybridized with oligonucleotide probes (design A), before washing, laser microdissection and amplification of oligonucleotide probes and genomic DNA. After separate library preparation from oligo probes and genomic DNA, samples were pooled and sequenced.
   **(a)** Genome browser tracks of raw sequencing data from oligonucleotide probes in 100NP samples. Blocks under genome browser sequencing read tracks represent the position of exons, where more abundant probes hybridize, as expected since introns are quickly spliced and degraded. The tracks show the number of probes mapping to the probe position on the probe reference map, all probe references are ordered in their linear genomic order. All four tracks were auto-scaled together. Three genes are highlighted, Ywhae, a house keeping gene, Oct4, which is specifically expressed in mESCs, and Gata4, which is specifically expressed in XEN cells.
   **(b)** Raw sequencing data from the parallel extraction and sequencing of cellular DNA. Tracks of genomic DNA from four GAM samples each produced from 1NP, two mESC samples and two XEN cell samples. The tracks show typical detection of genomic DNA that covers short consecutive regions of chromosomes, as expected from the DNA extracted from a thin nuclear slice.

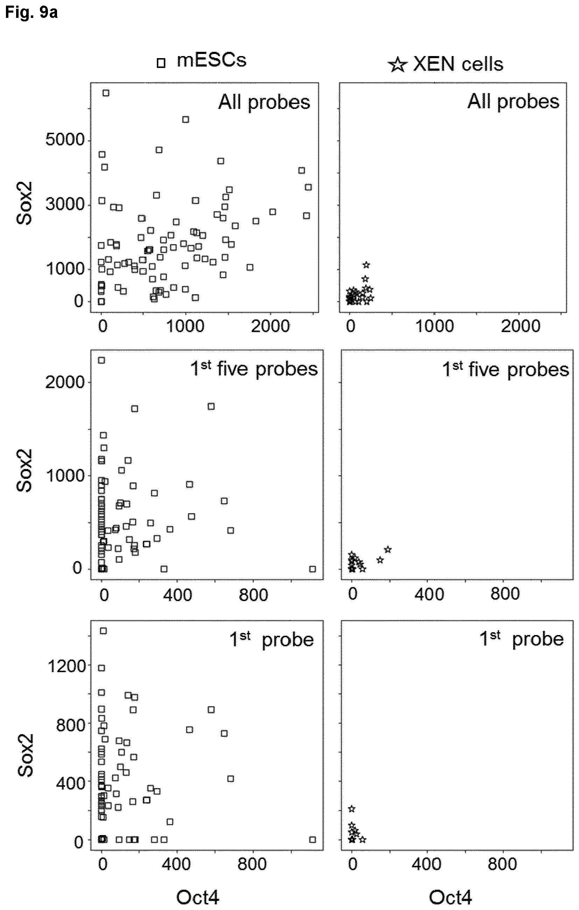

**Fig. 9 (a)** Expression of Sox2 and Oct4 in single ultrathin slices from mESC-F123 and XEN cells. Scatter plots show the relationship between the number of reads for Sox2 (Y-axis) and Oct4 (X-axis) sequenced from individual 1NP samples obtained from mESC-F123 (□ - left panels; number of samples= 96) and XEN cells (☆ - right panels; number of samples= 40) after oligo-seq analysis using oligo probe design A. The top panels represent the count of sequencing reads covering all probes across the gene, the middle panels represent the sequencing reads covering only the first five probes (from the TSS), and the bottom panels represent the sequencing reads covering only the first probe for each gene (from the TSS). Sox2 and Oct4 genes are expressed in mESCs and not in XEN cells. **(b)** Expression of Sox2 and Sox17 in single ultrathin slices from mESC-F123 and XEN cells. Scatter plots show the relationship between the number of reads for Sox2 (Y-axis) and Sox17 (X-axis) sequenced from individual 1NP samples obtained from mESC-F123 (□ - left panels; number of samples= (96) and XEN cells (☆ - right panels; number of samples= 40) after oligo-seq analysis using oligo probe design A. The top panels represent the count of sequencing reads covering all probes across the gene, the middle panels represent the sequencing reads covering only the first five probes (from the TSS), and the bottom panels represent the sequencing reads covering only the first probe for each gene (from the TSS). The Sox2 gene is expressed in mESCs but not in XEN cells, and the Sox17 gene is expressed in XEN cells but not mESCs.

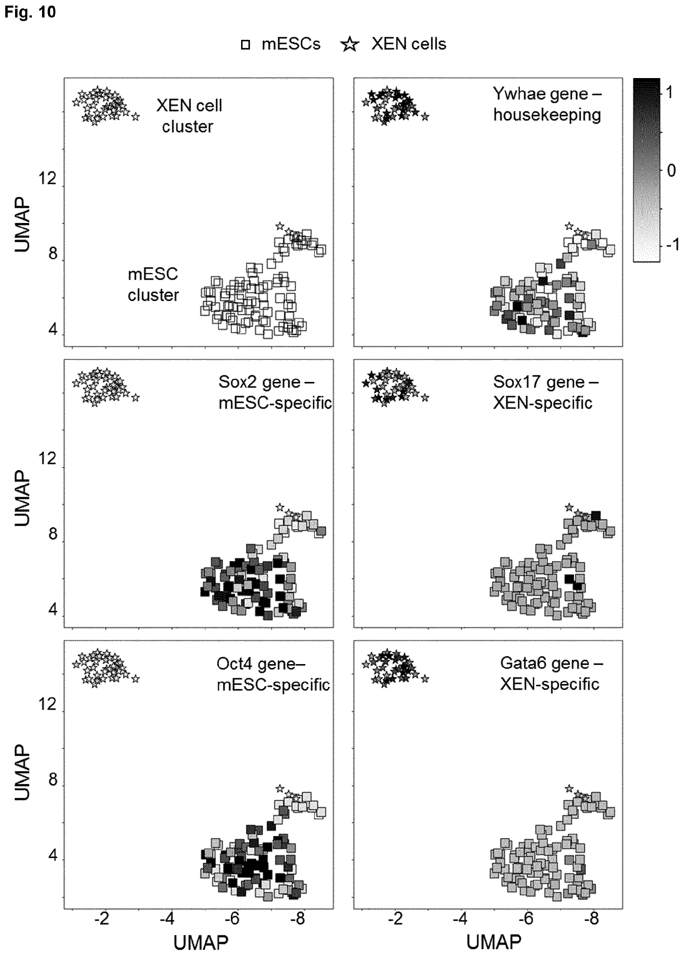

**Fig. 10****.** Oligo-seq in ultrathin cryosections distinguish different cell types. Clustering of mESC-F123 and XEN cells using expression data from single slice samples, and expression levels of transcripts encoded by specific genes in each cellular slice. UMAP clustering of all mESC-F123 (□) and XEN (☆) 1NP samples from their standardized read counts of probes targeting exons considering all targeted genes present in probe Design A, including non-expressed control genes. The top left panel shows the UMAP coordinates on the Y and X axis for all 1NP samples which clearly distinguish the slices from mESCs or XEN cells. The expression of genes Ywhae (housekeeping), Sox2 and Oct4 (mESC-specific), and Sox17 and Gata6 (XEN cell specific) are shown based of Z-score normalized expression (scale bar, Z-score of expression intensity represented in grey scale, top right).

### Examples

The method of the present invention, designated oligo-seq, detects the presence or abundance of nucleic acids, e.g., different RNA species in a tissue, cell or compartment, after stringent hybridization of short single-stranded oligonucleotides to said nucleic acids, followed by isolation of the hybridized oligos, and finally by their sequencing or PCR amplification. Oligonucleotides contain both a region of homology to the nucleic acid of interest and flanking regions of known sequence which are used for purposes of oligo amplification and detection, and for assignment to, e.g., an RNA species **(****Fig. 1****).** A more advanced implementation of oligo-seq combines RNA detection with Genome Architecture Mapping by recovering and sequencing the genomic DNA content of the compartment, in parallel with the extraction and sequencing of the RNA-hybridized oligo probes **(****Fig. 3b****).** Other implementations could involve tagging other types of probes (e.g. antibodies) with similar oligonucleotide sequences.

### Preferred probe structures

The central target region of each probe is unique to one individual target site within the mouse genome (mm10 assembly) and is approximately 35 bps long (homology region, HR; **Fig.** 1). Each target sequence is checked to exclude any potential for formation of secondary structures and has the thermal stability to withstand a 47°C stringent wash in 40% formamide.

On either side of the target region are sequence barcodes (**Fig**. 1). The 5' barcode (B1) is shared by all probes targeting a given gene, the 3' barcode (B2) is shared by all probes targeting exons or introns of the same gene. B1 and B2 allow for independent targeting of different genic regions either via FISH or qPCR. Barcodes are aligned to the whole genome sequence (mm10 assembly) to ensure that they do not exhibit complementarity and thus to avoid unspecific binding of the flanking regions to specific RNAs.

At the end of each probe there are two universal primer regions (UP1 and UP2; **Fig**. 1). Firstly, these enable amplification of probes from the original stock. Secondly, they allow to easily append Illumina compatible primers onto each oligo for sequencing. Additionally, the universal target regions can be used as target sites for FISH for a quick validation of successful probe hybridization in the compartment of interest during optimisation stages, a step which is however not essential to the invention.

In an alternative design, the probe structure could contain only the homology sequence to the RNA species of interest, and a 5' and/or a 3' universal primer sequence, without the barcode assigned to exons/introns. Probe structures can also contain one or more barcode sequences for certain target site properties, for compatibility with detection by FISH (for validation/ opmitisation purposes) and for PCR methods (for optimisation and as a standalone implementation). Probe structures can also include UMIs to reduce PCR biases and resolve to a probe abundance measure (**Fig**. 1).

### Proof of principle: detection of RNA

For the proof of principle library, we focused on a subset of 66 genes (**Fig**. 2). We chose key cell type markers that were appropriate for the cell types used in this implementation of the method. We aimed to test the power of oligo-seq to distinguish distinct cell types. This required testing whether 66 genes would be sufficient as well as determining the number of probes required per gene, the level of non-specific probe retention in the compartment, and the sensitivity to highly and lowly expressed genes. We chose to work with two developmentally close cell types with different gene expression profiles: mouse embryonic stem cells (mESC; cell line F123), and extraembryonic endoderm cells (XEN cell line). We choose probes specific to genes expressed in mESC or XEN cells. For instance, the Oct4 gene is expressed in mESCs and not in XEN cells, whereas the Gata4 gene is expressed in XEN cells and not in mESCs. To assess background retention of probes to non-expressed transcripts, we also included probes specific to neurons, and some specifically to dopaminergic neurons (e.g. Th, which encodes for Tyrosine hydroxylase). Both mESC and XEN cells actively divide. We therefore further chose to test for cell cycle markers, which are expressed in both cell types, but at varying levels in different cells of the dividing cell populations according to cell cycle stage. We also included probes against highly expressed and well researched IncRNAs that have preferential localizations in nuclear subcompartments and have roles in 3D architecture (Malat1, Firre, Neat1 and Xist). We also included probes against RNAs encoding pluripotency factors. For example, Nanog is a transcription factor expressed at varying levels within the population of mESCs and is not expressed in XEN cells. To explore the sensitivity of oligo-seq, we also included different numbers of probes for genes according to their lengths, e.g. 1100 probes (9.8% of all probes) were assigned to Satb2, a long neuronal gene not expressed in mESCs or XEN cells, and 17 probes to the rather short gene Sox2 which is expressed in mESCs and not XEN cells. Probes against housekeeping genes with different levels of expression were also included (genes Ywhae & Eif3h are highly expressed, and genes Faim & Alyref2 are expressed at a low level). All of the tested housekeeping genes exhibit uniform expression across a differentiation timeline between mESC to dopaminergic neurons (Ferrai et al., 2017, RNA polymerase II primes Polycomb-repressed developmental genes throughout terminal neuronal differentiation. Mol. Syst. Biol. 13, 946). Considering genes with different expression levels has the potential to help calibrate the expression of genes of interest, and provides a cell type independent control of successful experimental design.

All genes were targeted to their full extent including exons and introns, except for extremely long genes for which the number of probes targeting introns was reduced **(****Fig** .2). Accordingly, the number of probes targeting each gene was different from gene to gene, which was intentional in this proof-of-principle as a means to assess the relationship between the number of targeting probes per gene and our ability to detect expression as well as for retrieving the maximum amount of information from each gene. For example, we aimed to understand whether different regions of transcripts or sequence compositions of the target RNA region gave preferred specificity and sensitivity. We expect future implementations to use the minimum number of probes that provides robust detection potential, which may vary in different applications, e.g. a different number of probes or genes may be necessary to define the composition of cell types in a complex tissue, than to define the effects of a treatment or a disease in the expression profile of a cell type.

The library of oligonucleotides covering **66** genes of different lengths, expression patterns (e.g. only in mESCs or XEN cells, or in both, or none e.g. neuronal genes), and expression levels was selected based on published RNA-seq data for mESCs differentiated into neurons (Ferrai et al. 2017) **(****Fig. 2****).** For each gene, probes included oligonucleotides that covered exons or introns, except for intronless genes. In some cases, the exons of a gene (e.g., H3f3a) were covered by only a single oligonucleotide, whereas the exons of other genes such as Lhx1, were covered by 99 oligonucleotide probes. Similarly, the introns of genes were covered by different amounts of oligonucleotide probes. For instance, the introns of Hspa8 were covered by seven oligonucleotides, whereas the introns of Satb2 were covered by 1031 oligonucleotides. The neuronal gene Bdnf was covered by a total of 531 probes to investigate background and specificity of RNA detection in ESCs.

### Biological materials and ultrathin cryosectioning

As a proof-of-principle, we implemented oligo-seq using ultrathin cryosections from mESC and XEN cells. Thin nuclear cryosections were produced in the absence of resin-embedding, by a modified Tokuyasu method (Tokuyasu, K. T., 1973; Guillot 2004; Pombo et al., 1999). After fixation using electron-microscopy grade formaldehyde buffered with HEPES, cells were cryoprotected by embedding in a saturated sucrose solution followed by freezing in liquid nitrogen. Ultrathin cryosections of ∼220 nm thickness were cut in a Leica Ultracut cryomicrotome at -100°C and transferred to glass coverslips for validation of hybridization or to laser microdissection PEN slides for oligo-seq.

### Validation of oligo library hybridization by RNA-FISH on ultrathin cryosections

To test whether the oligo-seq probes efficiently hybridized to mouse ESCs, we used them first for fluorescence-based in situ hybridization (RNA-FISH) on ultrathin cryosections. Ultrathin cryosections are known to retain cellular RNA content in both nucleus and cytoplasm, while allowing for efficient hybridization of FITC-labelled oligo-dT probes in HeLa cells (Xie SQ and Pombo A., 2006, Distribution of different phosphorylated forms of RNA polymerase II in relation to Cajal and PML bodies in human cells: an ultrastructural study. Histochem. Cell Biol. 125, 21-31; Branco et al., 2006) and to provide efficient detection of single short RNAs, as the uPA/PLAU gene expression after activation in HepG2 cells (Ferrai et al., 2010).

After hybridization of the oligo-seq library to the cryosections from fixed and sucrose-embedded mESCs, unbound or partially hybridized probes were stringently washed and hybridized probes retained in the sections were revealed by a secondary hybridization with a fluorescently labelled, shorter ssDNA oligonucleotide with homology to the universal sequence of the flanking regions in the oligonucleotide probes (Beliveau et al., 2012). As expected, fluorescent signals were detected in the cytoplasm and nucleus, including in chromatin poor regions of the nucleus called interchromatin domains or splicing speckles (arrows; **Fig. 4****).** The specificity of oligo detection to hybridized RNA was confirmed by pretreatment of sections with RNAse **(****Fig. 4****, +RNAse),** and by the absence of probe signal in nucleoli (asterisk; **Fig. 4****),** which are rich in nascent and mature ribosomal RNAs, but not for the protein-coding transcripts that were targeted by the present probe library.

### Oligo-seg hybridization on cryosections

As a proof-of-principle, we combined oligo-Seq with GAM and used ultrathin cryosections from mESC and XEN cells. Oligonucleotide probes and cellular genomic DNA were extracted simultaneously, and oligo sequences were amplified (as described in **Fig. 3****)** in parallel with genomic DNA according to the GAM procedures (Beagrie et al. (2017); Beagrie et al., 2020, Multiplex-GAM: genome-wide identification of chromatin contacts yields insights not captured by Hi-C. bioRxiv 2020.07.31.230284; doi: https://doi.org/10.1101/ 2020.07.31.230284). As a first exploration of the presence or absence of cell-type specific transcripts in mESC or XEN cells, we started by using qPCR using primers homologous to the universal probe sequences, according to the procedure outlined in **Fig. 5b****.** Using laser microdissection (LMD) of cresyl-violet stained cryosections on LMD plastic coated slides, we collected several batches of approximately 100 single nuclear slices into single PCR tubes from (a) mESC-F123, (b) XEN cells, and (c) mESC-F123 cells pretreated using RNAse, as described in the methods. Genes specific to mESC (Sox2, Oct4) and XEN cells (Sox17, Gata6) were found more enriched (i.e. were detected with lower values of PCR cycle amplification) in their respective cell types **(****Fig. 6a****),** and showed equivalent enrichment for the highly-expressed Malat1 IncRNA which is expressed in both cell types. BDNF, a neuronal marker which is not expressed in either cell type, showed high numbers of PCR cycles in all samples analyzed, including in RNAse-treated mESCs. RNAse-treated mESC samples had no enrichment in cell-type specific gene signatures.
Next, we developed oligo-seq using NGS by producing libraries using the pipeline described in **(****Fig. 3b****).** We adapted an approach developed in-house for extraction and amplification of genomic DNA within the GAM pipeline. The adapted Malbac method is outlined in Winick-Ng, et al. 2020, which is based on the MALBAC whole genome amplification approach (Zhong C et al. 2012 Genome-Wide Detection of Single Nucleotide and Copy Number Variations of a Single Human Cell, Science, 338(6114): 1622-1626.) to the parallel detection of the oligo probes outlined in detail in the methods below.

In brief, to produce sequencing libraries from the probes from our samples, we designed primers that append sequences compatible with the Illumina Nextera kit onto the end of each oligo. These primers were directly incorporated into our in-house Whole Genome Amplification, such that probes and genomic DNA were PCR amplified simultaneously in the same reaction. Excess primers were digested with exonuclease I. Next, the PCR reaction containing both amplified oligo probes and genomic DNA was split in two, to allow that probe and genomic DNA from the same samples (containing 1NP or 100NPs) could be amplified independently **(****Fig. 3b****).** Probe and Genomic DNA sequencing libraries were indexed separately, DNA libraries were then pooled to be sequenced in the same run in a NextSeq Illumina sequencer (75bp read length, single end sequencing). We produced a total of 198 DNA libraries from mESCs, containing 96 genomic and 96 probe libraries, which originated from 96 samples each containing 1NP; and six DNA libraries, containing six genomic and six probe libraries, which originated from 6 samples each containing 100NPs (Table 4). We also produced similar libraries from mESCs treated with RNAse prior to oligo hybridization, and from XEN cells, as described in Table 4. After sequencing (2-4 million reads for genomic libraries, ∼500,000 reads per probe library), the reads were demultiplexed resulting in two separate Fastq files per sample, one for scRNA-GAM oligoprobes and one for the cellular genomic DNA. Detailed descriptions are outlined in the methods below.

Genomic sequencing files were mapped into the reference mouse genome (assembly mm10), and each sample had to pass quality control analyses of several parameters, especially percentage of orphan windows (<60%) and number of unique mappable reads (>25000). These parameters inform about incomplete extraction, failed laser microdissection or contamination which occur rarely (out of 154 1NP biological samples collected in the proof-of-principle experiment, 129 passed QC. Quality control can be performed, e.g., as described in Winick-Ng et al, 2020. Table 4 lists all samples collected. To map sequencing reads from the probe set, we built a probe reference sequence map that juxtaposed all probe sequences including probe specific barcodes and primers, keeping probes ordered by their genomic positioning in mm10. Low quality reads having poor mapping quality as determined by Bowtie2, i.e., that had low probability of mapping to one unique location, were removed. As each probe was sequenced from the same starting position (the 5' end) for a read length of 75bp into the RNA target homology sequence **(****Fig. 1****,** **Fig. 7****),** the raw data that obtained from oligo-seq was a discrete count of sequencing reads homologous to the probes per unique target site on the RNA species of interest. Simple summary statistics of RNA contact could then be applied based directly on read counts that aligned to the probe reference genome.

**Table 4. Proof-of-principle oligo-seq dataset collection**

| ***SAMPLE*** | ***1NP*** | ***100NP*** |
|---|---|---|
| ***mESC*** | *96* | *6* |
| ***mESC* + *RNAse*** | *26* | *2* |
| ***XEN*** | *40* | *2* |

Three oligo-seq collections were produced consisting of mESC-F123, RNAse-treated mESC-F123 and XEN, after laser microdissection of single nuclear profiles (1NP) or one hundred NPs (100NPs) into single PCR tubes (Table 4). 100NP samples were collected as "bulk" samples corresponding to a total cell material content of ∼3-5 cells in each sample. After mapping the different sets of samples containing 100NP mESCs or XEN cells to the probe reference map, we found clear evidence for detection of oligoprobes mapping to housekeeping genes (Ywhae) especially within exonic regions. This was expected since exons of expressed genes are present both at the site of transcription (in nascent RNAs) but also in the mature mRNA molecules that travel from nucleus to cytoplasm for translation into protein **(****Fig.8a****).** In contrast, intronic sequences were also detected throughout the intron of expressed genes, but at lower abundance since introns are typically short-lived and immediately degraded upon splicing of the nascent mRNA.

Next, we tested whether the oligo-seq mapping of RNAs in cryosections interfered with the detection of the genomic DNA, as in GAM. We found that the resulting read distribution from sequencing of 1NP samples showed the expected features **(****Fig.8b****),** with consecutive enriched patches of genomic DNA detection which varied between samples, as expected due to the sectioning of nuclei at random orientations. This observation was especially relevant as extraction and detection of genomic DNA from sub-cellular compartments, such as the thin (200nm thick) cryosections, can be prone to contamination, especially when biological samples are first put through additional procedures (such as oligo-Seq) before DNA extraction. However, we showed that combining GAM with oligo-seq can be successfully implemented, as the majority of samples collected (∼85% pass having >25,000 Uniquely mapped reads and <60% orphan windows) passed our current QC filtering steps for GAM data.

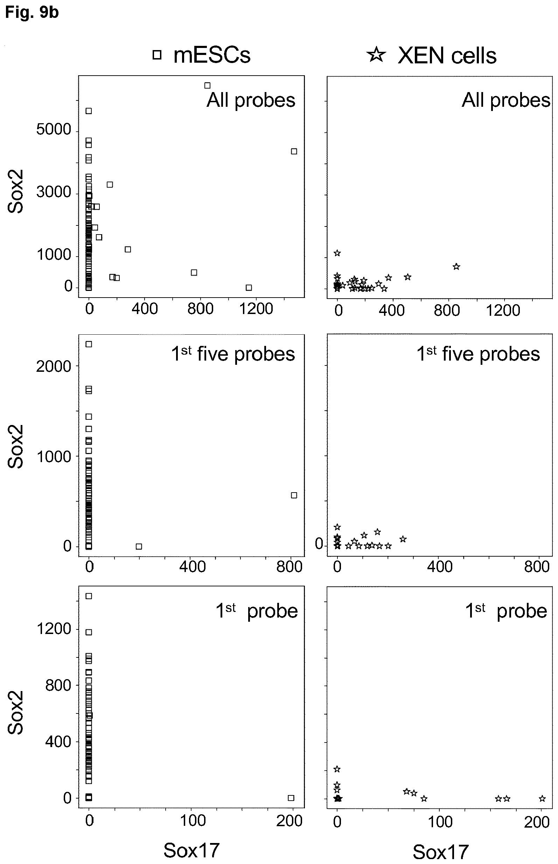

Enrichment for the cell-type-specific genes, Oct4 (mESCs) and Gata4 (XEN cells), was also evident when summing probe counts over the whole gene **(****Fig. 8a****).** For some genes, the first 5 probes from the transcription start site were enough to identify cell-type specific information **(****Fig.9a****).** For example, cryosections from mESCs hybridized with a high abundance of oligonucleotides complementary to Sox2 (mESC-specific) compared to oligonucleotides complementary to Sox17 (XEN-specific) and vice versa for Sox17 **(****Fig. 9b****).** The sum of probe counts for each gene per sample could successfully yield cell-type specific clusters of samples generated from mESC and XEN cell types, using UMAP (https://umap-learn.readthedocs.io/en/latest/basic_usage.html) showing that each sample was enriched for the transcripts expressed by its known cell type **(****Fig. 10****).** Clustering can be further improved by prior standardizing, exploring improved metrics of RNA abundance other than raw probe count per gene, and the potential filtering of poor-quality probes and samples based on the oligo-seq sequence.

From the present data, it is concluded that RNA abundance and gene expression information can be retrieved for each sample comprising a single thin slice from a single cell or one hundred such slices, using oligo-seq. Importantly, it is possible to unbiasedly cluster samples according to their cell type of origin, using two very close cell types from cell lines that represent the first lineage commitment of the embryo, based only on oligo-seq sequencing reads, which can potentially allow cell-type clustering from complex heterogenous tissue. One surprising outcome was the minimal number of probes required, approx. 5, for expression detection **(****Fig. 9a****,** bottom panels; **Fig. 9b****,** bottom panels).

As an independent application of oligo-seq with potential for extension into Droplet single-cell sequencing technologies, we tested the application of oligo-seq using cells in suspension. We collected whole cells (mESC, clone 46C) in solution, and cells were prepared according to the methods below, with the oligo-seq hybridization taking place in solution. After post-hybridization and stringent washes, cells were resuspended in PBS and approximately 1 to 20 mESCs were aliquoted separately into different PCR wells, and the presence of oligo probes for cell type specific genes was analysed by qPCR as detailed in the methods. As expected, mESCs showed an enrichment for mESC-expressed genes (Sox2 and Oct4) and for the ubiquitously expressed gene IncRNA Malat1 compared to the initial probe library composition (probes directly from stock). As expected, mESCs showed little detectable enrichment for oligoprobes specific for genes expressed in XEN cells (Sox17 and Gata6) and no enrichment for the neuronal marker Bdnf **(****Fig. 6b****).**

Flow cytometry techniques were incorporated to isolate single cells. Oligo-seq samples produced after hybridization of whole cells were equivalent to the ones produced from thin cryosections, and therefore could also be amplified for next-generation sequencing as they were hybridized, extracted and amplified for sequencing with the same oligo library and structure **(****Fig.1****),** as shown for the results shown above. Biological results from sequencing will also remain consistent.

### Materials and Methods

### Target site identification

Probe sequences that target gene bodies and exons are chosen compiled into their genomic sequences in a FASTA format. Genomic sequences can first be screened to exclude repetitive elements (http://repeatmasker.org/). Potential target sites can be filtered for homopolymeric runs, "N" bases, target site length, GC content and favourable and consistent melting temperature (Tm) predicted by nearest-neighbor dynamics (SantaLucia J, Jr (1998) A unified view of polymer, dumbbell, and oligonucleotide DNA nearest-neighbor thermodynamics. Proc Natl Acad Sci USA 95:1460-1465). All candidate sites passing these filters can be compiled into FASTQ format and can then be aligned against the whole genome of the target species to check that each candidate site is unique and does not align to multiple places of the genome; publicly available commonly used aligners for NGS data are Bowtie and BWA (Langmead B, Trapnell C, Pop M, Salzberg SL (2009) Bowtie: An ultrafast memory efficient short read aligner. Genome Biol 10:R25. 46; Langmead B, Salzberg SL (2012) Fast gapped-read alignment with Bowtie 2. Nat Methods 9:357-359. 47; Li H, Durbin R (2010) Fast and accurate long-read alignment with Burrows-Wheeler transform. Bioinformatics 26:589-595). NGS aligners can also inform on the mappability of the designed probe to the target site. A filtered and unique set of target sites can be checked using publicly available software to filter for secondary structures (Dirks RM, Pierce NA (2003) A partition function algorithm for nucleic acid secondary structure including pseudoknots. J Comput Chem 24:1664-1677), remove or concatenate overlapping target sites and overrepresented kmers (Marçais G, Kingsford C (2011) A fast, lock-free approach for efficient parallel counting of occurrences of k-mers. Bioinformatics 27:764-770). Target sites can be converted to their reverse complement to target either side of the DNA strand, necessary for targeting RNA. Target site sequence and genomic range can be formatted into standard BED format to allow ease of viewing of Genome browsers and data handling (Quinlan AR, Hall IM (2010) BEDTools: A flexible suite of utilities for comparing genomic features. Bioinformatics 26:841-842). Specificity of each oligo-seq probe is ultimately verified experimentally using appropriate controls (e.g. RNAse treatment or biological relevant tissue), and if partially or fully non-specific removed from the data analysis computationally. The ability to remove identified non-specific binding events at the discrete level of the oligo is a distinct advantage over other ISH techniques such as FISH and microarray-based technologies exemplified by Nanostring, which fail to discriminate between individual oligos in their detection methods.

### Probe Synthesis

ssDNA oligos were ordered from CustomArray, Inc. (Bothell, WA 98011, US). CustomArray provides libraries containing thousands of oligonucleotides, which are synthesized simultaneously using CMOS semiconductor technology.

Original stocks of oligo libraries were PCR amplified and stored safely as dsDNA libraries using primers complimentary to the universal primer ends which are present on all probes within the library UP1 and UP2 (Fig. 1). Oligo libraries were converted to ssDNA probe stocks for hybridization via a T7 amplification and a reverse transcription reaction as outlined in (Beliveau et al 2017). Alternative amplification methods are obtainable from, e.g.,https://oligopaints.hms.harvard.edu/protocols.

### Cells growth and fixation.

The mouse ES cells (mESCs) used for oligo-seq in thin cryosections were of the F123 line. The mouse ES cells (mESCs) used for the oligo-seq in whole cells in suspension were of the 46C line, a Sox1-GFP derivative of E14tg2a.

mESCs (clone F123) were cultured on a layer of feeder murine embryonic fibroblasts (MEFs), that had been mitotically inactivated (GSC-6201G, Global Stem). Feeder cells were grown at 37°C in feeder media (90 % DMEM (11995-065, Gibco), 10% FBS), and used up to 10 days after seeding. One day before culturing mESCs, dishes were coated with 0.1% gelatin and inactivated feeder cells were plated with a density of ∼1500 cells per mm². After feeders had settled (∼ 4 - 12 h after plating), mESCs were seeded onto the feeder layer and grown at 37°C in mESC-F123 media (DMEM (11995-065, Gibco), supplemented with 15% Knockout serum replacement (KSR, 10828028, Invitrogen), 1x Glutamax (35050, Gibco), 10 mM non-essential amino acids (11140-050, Gibco), 50µM beta-mercaptoethanol (31350010, Gibco), 1000 U/ml LIF (GFM200, Cell Guidance Systems). Cells were split onto new feeder-coated dishes every 48 h, and media was changed every 24 h. Typically, after two passages, feeder cells were removed from the mESC culture by splitting cells onto an uncoated dish for 30 mins. MEFs settle fast, while the mESCs remain in suspension. The cell suspension was transferred to a new uncoated plate for another 30 min to increase the efficiency of feeder removal, and afterwards cells were seeded on gelatine-coated dishes (ESGRO Complete Gelatine; SF008, Merck). The feeder-removal was repeated after 48 hours. mESCs were subsequently plated for harvest. As feeder-removal results in reduced levels of LIF in the culture, the LIF concentration in the media was doubled when the cells were in feeder-free culture conditions. Cells were harvested after ∼ 48 h at 70 - 80 % confluency.

mESCs, clone 46C, cells were grown at 37°C in a 5% (v/v) CO₂ incubator, in GMEM medium (Invitrogen, # 21710025), supplemented with 10% (v/v) fetal calf serum (FCS; PAA, # A15-151), 2 U/mL LIF (Millipore, # ESG1107), 0.1 mM β-mercaptoethanol (Invitrogen, # 31350-010), 2 mM L-glutamine (Invitrogen, # 25030- 024), 1 mM sodium pyruvate (Invitrogen, # 11360039), 1% penicillin-streptomycin (Invitrogen # 15140122), 1% MEM Non-Essential Amino Acids (Invitrogen, # 11140035) on gelatin-coated (0.1% (v/v)) Nunc T25 flasks. The medium was changed every day and cells were split every other day. Before sample collection, mESCs were plated on gelatin-coated (0.1% (v/v)) Nunc 10 cm dishes in serum-free ESGRO Complete Clonal Grade Medium (Millipore, # SF001-500), containing 1U/mL LIF. Cells were grown for 48h, with a medium change at 24h.

Murine extra-embryonic endoderm (XEN) cells are derived from primitive endoderm (Kunath et al., 2005, Imprinted X-inactivation in extra-embryonic endoderm cell lines from mouse blastocysts. Development 132, 1649-1661). XEN cells (clone IM8A1, Kunath et al., 2005) were cultured on 0.1% gelatin-coated surfaces in RPMI supplemented with 20% FCS, 2 mM L-glutamine, 1 mM sodium pyruvate, and 0.1 mM β-mercaptoethanol, at 37° C, in a 5% CO₂ incubator.

### Preparation of Cryosections

The cells were prepared for cryosectioning as described previously (Beagrie et al. 2017). Briefly, cells were fixed in 4% and 8% paraformaldehyde in 250 mM HEPES-NaOH (pH 7.6; 10 min and 2 h, respectively), pelleted, embedded (2 h) in 2.1 M sucrose in PBS and frozen in liquid nitrogen on copper stubs. Frozen cells may be stored in liquid nitrogen indefinitely. LMD 4 µm PEN membranes of metal-framed slides (Leica) were prepared by drawing a small rectangle of approximately 1x2 cm using a hydrophobic pen. Prior to cryosectioning, the glass knife used for cutting cryosections and the LMD PEN membrane for cryosection transfer were UV-treated (45 min, λ = 385 nm). Ultrathin cryosections were cut using an UltraCut UCT 52 ultracryomicrotome (Leica, Milton Keynes, UK) at around 220 nm thickness on a glass knife. Sections were captured in drops of RNAse-free 2.1M sucrose in PBS drops, held on a copper loop, and transferred to LMD 4 µm PEN membranes covered glass slides for laser microdissection (Leica, Milton Keynes, UK) or to glass coverslips for RNA-FISH.

### Oligo probe hybridization of cell/tissue cryosections on LMD PEN membranes

The hybridization oven for incubation was prepared by thoroughly cleaning the chamber and by introducing dampened blotting paper with sterile water, before being heated to 37°C (or between 30°C to 60°C, but ideally 37°C, the higher the temperature, the more the specificity is increased). All steps were carried out in RNAse-free conditions and all reagents were Molecular Biology grade.

Cryosections on LMD slides (for oligo-seq) or on glass coverslips (for RNA-FISH) were washed (3 times, 5 min each) in 0.2 µm filtered molecular-biology grade PBS, to wash off sucrose solution from cryosections. Cryosections were permeabilised (10 min) in 0.5% (V/V) Triton X-100 in PBS, followed by washing (3 times, 5 min each) in PBS.

For negative control samples, cryosections were treated with RNAse after the Triton X-100 treatment. After the second PBS wash from the previous step, cryosections were rinsed with 2x SSC, and incubated (2h, 37°C) in 250 microgram/mL RNAse A in 2xSSC, in a humidified chamber. The untreated sample was kept in 2x SSC at room temperature (about 20°C) or at 4°C.

Primary probe hybridization mixture (2x SSC, 30% formamide, 2 mM vanadyl ribonucleoside complex, 1 mg/mL yeast tRNA, 10% dextran sulfate, 0.1 µM primary probes) was denatured at 78°C (3 min), cooled on an ice block and kept at 4°C for a maximum of 4 h, until the permeabilization and RNAse treatments were completed.

Prior to hybridization with probe, cryosections were rinsed (2 times) with wash buffer (30% formamide, 2x SSC, 2 mM vanadyl ribonucleoside complex) only on the side of the PEN membrane containing the cryosections and incubated (5 min, room temperature) with wash buffer. Wash buffer was carefully removed and excess buffer was removed from the edges of the LMD slide using UV treated filter paper. Primary probe hybridization mixture (40 µL) was placed over the cryosection location on the LMD side. An RNAse-Free Hybrislip (Invitrogen) was overlaid carefully over the primary probe hybridization mixture and then sealed with rubber cement. To aid removal of the Hybrislip (Molecular Probes) post-hybridization, a generous amount of rubber cement was applied and extended to the metal rim of the slide. Samples were incubated (over 36h, or from 4-48h) in a humid chamber inside a hybridization oven at 37°C (or between 30°C to 60°C, ideally 37°C).

After hybridization, the rubber cement layer on the LMD slide was loosened by lightly covering it in wash buffer, and carefully removed along with the Hybrislip. Cryosections on the PEN membrane were quickly rinsed in wash buffer to avoid drying. Stringent washes, to remove excess primary probes, were performed (3 times, 20 min each) with wash buffer at 47°C (or between 37°C and 65°C, ideally 47°C; increased temperature increases specificity). Wash buffer was removed by washing (3 times, 5 min each) in PBS. LMD slides were rinsed 1 time with water, and incubated (20 min) in 1% cresyl violet in water. Excess cresyl violet was removed by rinsing the top and reverse of the LMD slides with water (3 times each), and allowed to dry before immediately proceeding to the LMD (storage of hybridized samples prior to laser microdissection may be possible, for example in PBS at 4°C, though storage is currently not tested).

### Isolation of Nuclear Profiles

Individual NPs were isolated from the cryosection by laser microdissection using a Leica laser microdissection microscope (Leica Microsystems, LMD7000) using a 63x dry objective. Slices from individual cells were identified under bright-field imaging and the laser was used to cut the slide membrane surrounding each cell. Cut cell slices were collected into PCR adhesive caps (AdhesiveStrip 8C opaque; Carl Zeiss Microscopy #415190-9161-000). In each plate collection, two empty membrane regions with areas equivalent to 100NPs or 1NPs were collected as negative controls. These negative controls were also used to make sequencing libraries for quality control purposes. Laser microdissected samples on AdhesiveStrip caps were stored at -20°C until further use.

### WGA

**GAT-7N:** GTGAGTGATGGTTGAGGTAGTGTGGAGNNNNNNN (SEQ ID NO: 1)
**GAT-COM:** GTGAGTGATGGTTGAGGTAGTGTGGAG (SEQ ID NO: 2)
**Probe primer Forward:** GGCACAACGTTGCAGCACAG (SEQ ID NO: 3)
**Probe primer Reverse:** CACCAACGCTACCAGCTCCG (SEQ ID NO: 4)
**Probe Primer A MeRev Forward:**
   TCGTCGGCAGCGTCAGATGTGTATAAGAGACAGGGCACAACGTTGCAGCACAG(SEQ ID NO: 5)
**Probe Primer B MeRev Reverse:**

GTCTCGTGGGCTCGGAGATGTGTATAAGAGACAGCACCAACGCTACCAGCTCCG (SE
Q ID NO: 6)

**Lysis Mix:** (21 mM Tris-HCI pH=8.0, 1.4 nM EDTA pH=8, Guanidinium-HCI pH=8.5, 3.5% Tween 20, 0.35%Triton X-100, 123 µg of Qiagen protease (19157), Total volume 10µl

**2X DeepVent buffer:** 2x Thermo Pol Reaction Buffer (B9004s), 4 mM MgSO₄ (B1003s), 400 µM dNTPs (N0447L)

**PCR mix 1:** 1.333x DeepVent buffer, 0.666 µM GAT-7n, 0.0066 µM Probe primer forward, 0.0066 µM Probe primer reverse, 80 U/mL DeepVent Polymerase

**PCR mix 2:** 1x DeepVent buffer, 1.2 µM GAT-COM, 0.12 µM Probe primer forward, 0.12 µM Probe primer reverse, 60 U/mL DeepVent polymerase, 0.4 mM dNTPs.

**Probe PCR mix:** 1x DeepVent buffer, 1 µM Probe primer forward, 1 µM Probe primer reverse, 30 U/mL DeepVent polymerase

**Genomic PCR mix:** 1x DeepVent buffer, 1 µM GAT-COM, 30 U/mL DeepVent polymerase

Lysis mix (10 µL) was added to each well of a 96-well plate. The 96-well plate was closed with LMD caps containing laser-microdissected samples and tightly sealed. The 96-well plate was inverted and lysis mix was collected in the lid of the cap by gently spinning the inverted plate at 200xg for 2 min. Samples were incubated overnight at 60°C to digest the sample.

The following day the 96-well plate buffer was collected at the bottom of the wells by centrifuging for 2 min at 200xg. The protease was heat inactivated at 75°C for 30 min in a PCR machine.

For linear amplification of genomic DNA and simultaneous probe amplification, 30 µL of PCR mix1 was added to each well and incubated as follows: 95°C - 5 min, 11x (60°C - 50 s, 20°C - 50 s, 30°C - 50 s, 40°C - 45 s, 50°C - 45 s, 65°C - 7 min, 95°C - 20 s), 72°C - 5 min. It is noted that, in general, in the method of the invention, the concentrations of primer for this step can be extraordinarily low, e.g., less than 0.001 µM forward and reverse primer each.

For simultaneous amplification of genomic DNA and probes 20 µL of PCR mix 2 was added to each well. This was incubated as follows [95°C - 3 min, 3x (95°C - 20 s, 58°C - 30 s, 72°C - 3 min), 72°C - 3 min].

Excess primers were removed by adding 5 µL of exonuclease solution [1x Exonuclease buffer (M0293L), 10 U Exonuclease1 (M0293L)] to each well and incubated at 37°C for 40 min followed by heat-inactivation of exonuclease for 72°C for 20 min.

To PCR-amplify probes, 30 µL of reaction mix from each well was transferred to a new 96 well plate, 20 µL of Probe primer mix was added to each well of the new 96-well plate and incubated as follows, 95°C - 3 min, 18x (95°C - 20 s, 60°C - 30 s, 72°C - 1 min) 72°C - 3 min. In parallel, to PCR-amplify Genomic DNA, 20 µL of Genomic PCR mix was added to each well in the original plate containing approximately 30 µL of original reaction mix, and incubated as follows: 95°C - 3 min, 24x (95°C - 20 s, 58°C - 30 s, 72°C - 3 min), 72°C - 3 min). Plates were kept at -20°C until further use.

### Through to sequencing:

The Genomic DNA plate and Probe DNA plates were cleaned in 1.7x and 1.8x SPRI beads and eluted in 20 µL of ultra-pure water (Sigma). DNA concentrations were measured with Quant-iT Picogreen dsDNA quantification assay.

Genomic DNA concentration in each sample was normalized to 1 ng/µL concentration. Genomic DNA was indexed and made into libraries using an in-house Tn5 library preparation protocol directly compatible with Illumina systems (Winick-Ng, W., et al., 2020, Cell-type specialization in the brain is encoded by specific long-range chromatin topologies, Biorxiv. https://doi.org/10.1101/2020.04.02.02099). Briefly, a reaction mix containing (250mM TAPS-HCI pH 8.5, 10% PEG, 250 µM MgCI2, 1 ng Genomic DNA) to a total of 5 µL, the tagmentation mix was incubated for 55°C for 7 min for the transposition reaction.

Tn5 was heat inactivated by a further incubation at 70°C for 5 min. Genomic DNA in 96-well plates was indexed with primers corresponding to Set A I5 and I7 Illumina barcodes from the Nextera XT system. PCR mix (5 µL) was directly added to the transposed DNA (The PCR mix consisted of 2.5x KAPA GC buffer (KB2501), 0.9 mM dNTPs (KN1009), 0.06 U/µL KAPA HiFi polymerase (KE2004), 2.5 µM i5 primer, 2.5 µM i7 primer). The PCR was incubated as follows (95°C - 30 s, 12 x (95°C - 10 s, 55°C - 30 s, 72°C - 30 s), 72°C - 5 min).

DNA amplified from the oligo probes in the mirror 96-well PCR plates was directly indexed by PCR by appending on Illumina Set B I5 I7 Set B indexes using the KAPA Biosystems HiFi kit. The PCR mix consisted of 1x KAPA GC buffer (KB2501), 0.45 mM dNTPs (KN1009), 0.03 U/µL KAPA HiFi polymerase (KE2004), 1.25 µM i5 primer, 1.25 µM i7 primer) and 1 µL of probe DNA sample to a total of 10 µL. The PCR reaction was run as follows (95°C - 30 seconds, 9x (95°C - 20 s, 60°C - 30 s, 72°C - 2 min), 72°C - 5 min).

The indexed genomic DNA plate and probe DNA plate were cleaned in 1.7x SPRI beads and eluted in 20 µL ultra-pure water (Sigma). DNA concentrations were measured with Quant-iT Picogreen dsDNA quantification assay.

The genomic DNA library samples were combined at equal concentration (96 libraries total). Separately, the probe samples were also combined at equal concentration (96 libraries total). Each pooled sequenced library was cleaned two times in a 1.6x SPRI bead clean up and eluted in 50 µL ultra-pure water. Concentrations of the combined pools were determined by Qubit high sensitivity dsDNA assay. Average fragment sizes were determined by BioAnalyzer. Combined genomic and probe libraries were then pooled together at equal molarity prior to sequencing. The libraries were sequenced (75bps single-end) together in a single high-throughput sequencing run in an Illumina NextSeq500 sequencer.

### Mapping probe and genomic DNA

Sequenced reads from each Genomic DNA library were mapped to the mouse genome assembly GRCm38 (Dec. 2011, mm10) with Bowtie2 using default settings. All non-uniquely mapped reads, reads with mapping quality < 20 and PCR duplicates were removed and excluded from further analyses.

Sequenced reads from each Genomic DNA library were mapped to the known probe sequences with Bowtie2 using default settings. All non-uniquely mapped reads and reads with mapping quality < 20 were removed and excluded from further analyses. The count for each probe was defined for each gene as the number of reads within the first 75bps of each read. Bedtools 'Map' function was used to count each probe in each position.

### PCR-based detection of ssDNA

Nuclear cryosections were stored in 4 mm AdhesiveStrip 8C opaque ZEISS LMD caps at - 20°C until further use.

10 µL of lysis mix was added to each sample well of a 96-well plate. The plate was closed with LMD caps containing cryosections and tightly sealed. The 96-well plate was inverted and lysis mix was collected in the lid of the cap by gently spinning the inverted plate at 200xg for 2 min. Samples were incubated overnight at 60°C.

The following day the 96 well plate buffer was collected at the bottom of the wells by centrifuging for 2 min at 200xg. The protease was heat inactivated at 75°C for 30 min in a PCR machine.

30 µL of PCR mix (20 µL 2x DeepVent polymerase, 0.16 µL forward primer, 0.16 µL reverse primer, 1.8 µL 100U/ml DeepVent polymerase) was added to each well. This was amplified for (95°C - 3 min, 30x (95°C - 20 s, 58 °C - 30 s, 72°C - 1 min), 72°C - 3 min). PCR mix was cleaned in 1.8x SPRI beads and concentrations determined by Qubit hsDNA assay. All samples were normalized to 0.2 ng/µL. qPCR was mix made as follows (2.5 µL normalized sample, 12.5 µL 2xSybr-Green PCR master mix, 0.75 µL 10 µM Primer stock (containing both forward and reverse primer), PCR-reaction volume adjusted with 25 µL water, Molecular Biology grade). The qPCR was run as follows: 95°C - 5 min, 40x (95°C - 30 s, 60°C- 15 s, 72°C - 30 s).

### Fluorescence-based detection of ssDNA as means of validation and optimization

For RNA-FISH using the oligo-seq probe, we used the same stock of primary probe and hybridization conditions. To visualize the hybridized oligo-seq probes on a fluorescence microscope, cryosections were hybridized with bridge and secondary probes (Fig. 4b).
Primary: [Universal primer 1][Barcode 1][Target homology][Barcode 2][Universal Primer 2]
Bridge: [Barcode homology][TT hinge][Secondary homology]
Secondary: [AlexaFluor647][AA][Bridge homology][AA][AlexaFlour647]

Cryosections (220nm thick) from mESC-F123 frozen samples were transferred to glass coverslips (1cm diameter), washed (3 times, 5 min each) in PBS, permeabilised (10 min) in 0.1% (V/V) Triton X-100 in PBS, washed (3 times, 5 min each) in PBS. Samples were rinsed in 2xSSC and stored in 2xSSC at 4°C for 2h, or incubated (2h, 37°C) in 250 µg/ml RNAse A in 2xSSC, in a humidified chamber.

Primary probe hybridization mixture (2x SSC, 30% formamide, 2 mM vanadyl ribonucleoside complex, 1 mg/mL yeast tRNA, 10% dextran sulfate, 0.1 µM primary probes) was denatured at 78°C (3 min), cooled on an ice block and kept at 4°C for a maximum of 4h, until cryosections were ready.

Prior to hybridization with probes, cryosections were rinsed (2 times) with wash buffer (30% formamide, 2x SSC, 2 mM vanadyl ribonucleoside complex) and incubated (5 min, room temperature) with wash buffer. Wash buffer was carefully removed and excess buffer was removed from the edges of the coverslip using UV-treated filter paper. Primary probe hybridization mixture (8 µL) was placed on an RNAse-free Hybrislip (Invitrogen) and the coverslip was overlaid carefully over the primary probe hybridization mixture and then sealed with rubber cement. Samples were incubated (over 36h (two overnights), or from 4-48h) in a humid chamber inside hybridization oven at 37°C (or between 30°C to 60°C, ideally 37°C).

Rubber cement was loosened by slightly covering it with wash buffer. Subsequently, the rubber cement was carefully removed. Cryosections were quickly rinsed in wash buffer to avoid drying. Stringent washes, to remove excess primary probes, were performed (3 times, 20 min each) with wash buffer at 47°C (or between 37°C and 65°C, ideally 47°C; increased temperature increases specificity).

Wash buffer was removed by washing (3 times) in 2x SSC. Excess buffer was removed carefully with UV-treated filter paper. Secondary hybridization buffer (8 µL) was placed on parafilm protected from UV light, the side of the coverslip containing cryosections was incubated on secondary hybridization buffer containing (30% formamide, 2X SSC, 1.6 µM secondary oligos, 1.4 µM bridge oligos). Stringent washes to remove excess secondary oligos were performed in 2x SSC, 40% formamide (3 times, 5 min each) at room temperature, with mild shaking. Coverslips were then washed in 2x SSC for 5 min. Coverslips were finally washed in 1x PBS (3 times, 5 min each) and either stored at 4°C or mounted in DAPI-Vectashield and imaged on a Leica confocal microscope. Images from cryosections were acquired on a confocal laser-scanning microscope (Leica TCS SP8; 63x objective, NA 1.4) equipped with a 405nm diode, and a white-light laser, using pinhole equivalent to 1 Airy disk. Images from the different channels were collected sequentially to avoid fluorescence bleed-through.

### In solution oligo-seq details

mESCs (clone 46C) were plated (4.5x10⁶ cells) in Nunc T75 flasks and grown for 48h w/ mESC-46C Media +LIF. Cells were trypsinised (2 min at 37°C) with 0.05% trypsin in PBS (2.5 mL each in two Nunc T75 flasks). Trypsinization was quenched with 10 ml of mESC-46C culture media without LIF. Content of two flasks was then pooled into a 50 mL conical flask (25 mL of single cell suspension) and centrifuged for 3 min at 280x*g*. Cells were counted (1:10 dilution in PBS) with a Scepter^{™} 2.0 Handheld Automated Cell Counter. Supernatant was removed and cells were resuspended at a concentration of 5x10⁶ cells/mL in mESC-46C culture media plus LIF. Cells were aliquoted into 1.5 mL tubes at 5x10⁶ cells per tube.

Trypsin-detached mESCs were pelleted at 900xg for 10 min at 4°C. Cells were then washed with 1x PBS and resuspended in 500 µL of 1x PBS. Cells were fixed with 4% electron-microscopy grade formaldehyde in PBS (1 mL) for 20 min at room temperature by adding 500 µL of 8% formaldehyde in 1x PBS on a rotating wheel at RT. Cells were pelleted at 300x*g* for 6 min at 4°C and rinsed and washed with 1xPBS for 5 mins. Cells were pelleted at 300x*g* for 6 minutes at 4°C to re-pellet cells. Cells were resuspended and permeabilized with 200 µL of 0.5% Triton X-100 in PBS for 10 min on ice with mild shaking. 700 µL of PBS was added and cells were pelleted at 300x*g* for 6 min at 4°C. During this step, primary probes were denatured at 78°C for 3 min directly moved to an ice block for rapid cooling to avoid renaturation.

Cells were resuspended and incubated for 5 min with mild shaking in 1x wash buffer (30% formamide, 2xSSC, 2mM vanadyl ribonucleoside complex) and centrifuged at 300x*g* for 6 min at 4°C. All buffer was carefully removed and cells were gently resuspended in 100 µL of primary probe hybridization mixture (30% formamide, 2xSSC, 2mM vanadyl ribonucleoside complex, 0.1µM primary probe, 1mg/ml yeast tRNA, 10% dextran sulfate) and incubated overnight at 37°C in a humidified chamber mildly rotating.

900 µL of 1x EWB was directly added to the 1.5 mL tube and pelleted at 500x*g* for 6 min. Supernatant was removed and cells resuspended in 500 µL of 1x EWB and washed for 20 min at 47°C. Cells were pelleted at 2000xg for 3 min, supernatant removed and resuspended in 500 µL 1x EWB and washed for 20 min at 47°C. Cells were pelleted at 2000xg for 3 min and rinsed and washed in 1x PBS (2 washes 5 min each). Cells were resuspended in 50 µL of 1x PBS. Approximately 1-20 cells were aliquoted in a 5 µL solution of PBS into separate wells of 96-well plate and stored at -20°C until further use.

17 µL of lysis buffer (30 mM Tris-HCI pH 8.0, 2 mM EDTA pH 8.0, 0.8 M Guanidinium-HCI pH 8.5, 5% Tween-20, 0.5% Triton X-100) and 3 µL of Qiagen Protease (Cat 19157) was added to each sample well. Samples were incubated overnight at 60°C with orbital shaking. Protease was heat inactivated the following morning at 75°C for 30 min. 40 µL of PCR mix (2x DeepVent Solution, 0.24 µL of 100 µM Forward primer, 0.24 µL 100 µM Reverse primer, 1.8 µL DeepVent Polymerase, 8 µL ultra-pure water) was added to each well. The PCR was run as follows: 95°C - 3 min, 30x (95°C - 20 s, 58 - 30 s, 72°C - 1 min), 72°C - 3 min).

PCR mix was cleaned in 1.8x SPRI beads and concentrations were determined by perfoming the Qubit hsDNA assay. All samples were normalized to 0.2 ng/µL. qPCR mix was made as follows (2.5 µL normalized sample (0.5 ng total), 12.5 µL Sybr-Green [TS1], 0.75µL 10 µM Primer Stock (containing both forward and reverse primers for gene of interest), PCR-Clean water to 25 µL).The qPCR was run as follows: 95°C - 5 min, 40x (95°C - 30 s, 60°C - 15 s, 72°C - 30 s, plate read for Sybr-green).

### References

1. Josefsen, K. and Nielsen, H. (2011) Northern Blotting Analysis. Methods Mol Biol. 703, 87-105.
2. https://www.thermofisher.com/de/de/home/references/ambion-tech-support/ribonuclease-protection-assays/general-articles/the-basics-what-is-a-nuclease-protection-assay.html).
3. Miller, M.B. and Tang, Y.-W. (2009) Basic Concepts of Microarrays and Potential Applications in Clinical Microbiology., Clin Microbiol Rev. 22(4): 611-633.
4. https://www.illumina.com/documents/products/techspotlights/techspotlight_sequencing.p df
5. Stark R., Grzelak, M. and Hadfield, J. (2019) RNA sequencing: the teenage years. Nature Reviews Genetics 20, 631-656.
6. Ziegenhain C., Vieth, B., Parekh, S., Reinius, B., Guillaumet-Adkins, A., Smets, M., Leonhardt, H., Heyn, H., Hellmann, I. and Enard, W. (2017) Comparative Analysis of Single-Cell RNA Sequencing Methods. Mol Cell. 85(4), 631-643.
7. Beliveau, B.J., Joyce, E.F., Apostolopolous, N., Yilmaz, F., Fonseka, C.Y., McCole, R.B., Chang, Y., Li, J.B., Senaratne, T., N., Williams, B.R., Rouillard, J.M. and Wu, C.T. (2012) Versatile design and synthesis platform for visualizing genomes with Oligopaint FISH probes. Proc Natl Acad Sci 109(52), 21301-6.
8. Geiss GK, Bumgarner RE, Birditt B, et al. (2008) Direct multiplexed measurement of gene expres-sion with color-coded probe pairs. Nat Biotechnol. 26(3), 317-325.
9. Stahl PL, Salmén F, Vickovic S et al. (2016) Visualization and analysis of gene expression in tissue sections by spatial transcriptomics. Science 353(6294), 78-82.
10. WO2019/157445
11. Merritt et al. (2020) Multiplex digital spatial profiling of proteins and RNA in fixed tissue. Nature Biotechnology 38, 586-599.
12. Marshal et al. (2020) HyPR-seq: Single-cell quantification of chosen RNAs via hybridization and sequencing of DNA probes. BioRxiv preprint doi: https://doi.org/10.1101/2020.06.01.128314).
13. Tropini et al. (2017) The Gut Microbiome: Connecting Spatial Organization to Function. Cell Host & Microbe 21(4), 433-442.
14. Liu et al. (2017) Low-abundant species facilitates specific spatial organization that promotes multispecies biofilm formation. Environ Microbiol. 19, 2893-905.
15. Liu et al. (2019) Deciphering links between bacterial interactions and spatial organization in multispecies biofilms. The ISME Journal 13, 3054-3066.
16. Tokuyasu, K. T. (1973) A technique for ultracryotomy of cell suspensions and tissues. , J. Cell Biol. 57, 551-65.
17. Guillot P.V., Xie S.Q., Hollinshead M., Pombo A. (2004) Fixation-induced redistribution of hyperphosphorylated RNA polymerase II in the nucleus of human cells. Exp. Cell Res. 295, 460-468.
18. Pombo A, Hollinshead M, Cook PR (1999) Bridging the resolution gap: Imaging the same transcription factories in cryosections by light and electron microscopy. J. Histochem. Cytochem. 47, 471-480.
19. McDowall et al. (1989) The structure of organelles of the endocytic pathway in hydrated cryosections of cultured cells, Eur. J. Cell Biol. 49, 281 - 294.
20. Chen et al. (2014) Nano-Dissection and Sequencing of DNA at Single Sub-Nuclear Structures. Small 10:3267.
21. Lučič V., et al. (2013) Cryo-electron tomography: The challenge of doing structural biology in situ. J Cell Biol 202 (3), 407.
22. https://www.protocols.io/view/Stellaris-RNA-FISH-Protocol-for-FrozenTissue-iwgs5v
23. https://www.protocols.io/view/exfish-tissue-slice-n6adhae
24. Branco, M. R. & Pombo, A. (2006) Intermingling of chromosome territories in interphase suggests role in translocations and transcription-dependent associations. PLoS Biol. 4, e138.
25. Xie, S.Q. et al. (2006) Splicing speckles are not reservoirs of RNA polymerase II, but contain an inactive form, phosphorylated on Serine2 residues of the C-terminal domain. Mol. Biol. Cell 17, 1723-1733.
26. Branco, M. R. (2006) Correlative microscopy using Tokuyasu cryosections: applications for immunogold labelling and in situ hybridisation. Cell Imaging (Methods Express Series)", ed. D. Stephens, Scion Publishing Ltd. (Bloxham, UK), 201-217.
27. Ferrai, C., et al. (2010) Poised transcription factories prime silent uPA genes prior to activation. PLoS Biology 8, e1000270.
28. Macosko et al. (2015) Highly Parallel Genome-wide Expression Profiling of Individual Cells Using Nanoliter Droplets. Cell 161(5), 1202-1214.
29. Levsky, J.M. et al. (2002) Single-cell gene expression profiling. Science 297, 836-840.
30. Tripathi et al., 2015, RNA Fluorescence In Situ Hybridization in Cultured Mammalian Cells. In: Carmichael G. (eds) Regulatory Non-Coding RNAs. Methods in Molecular Biology (Methods and Protocols), vol 1206. Humana Press, New York, NY. https://doi.org/10.1007/978-1-4939-1369-5_11.
31. Pombo, A. (2003) Cellular genomics: which genes are transcribed when and where? Trends Biochem. Sci. 28, 6-9.
32. Winick-Ng, W., et al., 2020, Cell-type specialization in the brain is encoded by specific long-range chromatin topologies, Biorxiv. https://doi.org/10.1101/2020.04.02.020990.
33. Möller et al. (2012) Proteomic analysis of mitotic RNA polymerase II complexes reveals novel in-teractors and association with proteins dysfunctional in disease. Mol. Cell. Proteomics 11(6):M111.011767.
34. https://www.protocols.io/view/Stellaris-RNA-FISH-Protocol-for-FrozenTissue-iwgs5v
35. https://www.protocols.io/view/exfish-tissue-slice-n6adhae
36. Domiguez and Kolodney (2005) Wild-type blocking polymerase chain reaction for detection of single nucleotide minority mutations from clinical specimens. Oncogene 24, 6830-6834.
37. Pombo, A., et al. (1994) Adenovirus replication and transcription sites are spatially separated in the nucleus of infected cells. EMBO J. 13(12), 5075-5085.
38. Femino, A.M., et al. (1998) Visualization of Single RNA Transcripts in Situ. Science, 280(5363).
39. Beliveau et al. (2017) In situ super-resolution imaging of genomic DNA with OligoSTORM and OligoDNA-PAINT. Methods Mol Biol. 1663, 231-252.
40. https://oligopaints.hms.harvard.edu/protocols
41. Dhanoa, J. K., Sethi, R. S., Verma, R., Arora, J. S., & Mukhopadhyay, C. S. (2018). Long non-coding RNA: its evolutionary relics and biological implications in mammals: a review. Journal of animal science and technology, 60, 25.
42. Catalanotto, C., Cogoni, C., & Zardo, G. (2016) MicroRNA in Control of Gene Expression: An Overview of Nuclear Functions. International journal of molecular sciences, 17(10), 1712.
43. van Heesch et al. (2019) The Translational Landscape of the Human Heart. Cell 178(1), 242-260.
44. Arnold et al., (2020) Diversity and Emerging Roles of Enhancer RNA in Regulation of Gene Ex-pression and Cell Fate. Front. Cell Dev. Biol. 7.
45. Kristensen et al. (2019) The biogenesis, biology and characterization of circular RNAs. Nat Rev Genet. 20(11), 675-691.
46. Akhter et al. (2018) Circular RNA and Alzheimer's Disease. Adv Exp Med Biol. 2018;1087:239-243.
47. Michelini, F., Pitchiaya, S., Vitelli, V. et al. (2017) Damage-induced IncRNAs control the DNA dam-age response through interaction with DDRNAs at individual double-strand breaks. Nat Cell Biol 19, 1400-1411.
48. Lloret-Llinares et al. (2016) Relationships between PROMPT and gene expression. RNA Biol. 13(1), 6-14.
49. Feng et al., 1995, The RNA component of human telomerase. Science 269(5228), 1236-1241.
50. Weibel, E.R. (1979) Stereological Methods: Practical Methods for Biological Morphometry. Vol. 1 Academic Press, London, UK.
51. Weibel, E.R. (1980) Stereological Methods: Theoretical Foundations. Vol. 2. Academic Press, London, UK.
52. Mateo et al. (2019) Visualizing DNA folding and RNA in embryos at single-cell resolution. Nature 568, 49-54.
53. Zhao, N., et al. (2019) A Genetically Encoded Probe for Imaging Nascent and Mature HA-tagged Proteins in Vivo. Nat. Commun. 10(10) 2947.
54. Beagrie et al. (2017). Complex multi-enhancer contacts captured by genome architecture mapping. Nature. 543, 519-524.
55. Markowski et al. (2020) GAMIBHEAR: whole-genome haplotype reconstruction from Genome Architecture Mapping data. bioRxiv 2020.01.30.927061.
56. van Buggenum et al. (2018) Immuno-detection by sequencing enables large-scale high-dimensional phenotyping in cells. Nature Communications.
57. Buenrostro, J. D., et al. (2015) ATAC-seq: A Method for Assay-ing Chromatin Accessibility Genome-Wide. Current protocols in molecular biology, 109, 21.29.1-21.29.9.
58. WO 2016156469
59. Beliveau et al. (2018) OligoMiner provides a rapid, flexible environment for the design of genome-scale oligonucleotide in situ hybridization probes. PNAS 115(10), 183-192
60. GeoMx^{™} product brochure, available on https://www.nanostring.com/products/geomx-digital-spatial-profiler/geomx-dsp
61. Ferrai et al. (2017) RNA polymerase II primes Polycomb-repressed developmental genes throughout terminal neuronal differentiation. Mol. Syst. Biol. 13, 946.
62. Xie SQ and Pombo A. (2006) Distribution of different phosphorylated forms of RNA polymerase II in relation to Cajal and PML bodies in human cells: an ultrastructural study. Histochem. Cell Biol. 125, 21-31.
63. Beagrie et al. (2020) Multiplex-GAM: genome-wide identification of chromatin contacts yields insights not captured by Hi-C. bioRxiv 2020.07.31.230284; doi: https://doi.org/10.1101/ 2020.07.31.230284.
64. Kunath et al. (2005) Imprinted X-inactivation in extra-embryonic endoderm cell lines from mouse blastocysts. Development 132, 1649-1661.
65. http://repeatmasker.org/
66. SantaLucia J, Jr (1998) A unified view of polymer, dumbbell, and oligonucleotide DNA nearest-neighbor thermodynamics. Proc Natl Acad Sci USA 95:1460-1465.
67. Langmead B, Trapnell C, Pop M, Salzberg SL (2009) Bowtie: An ultrafast memoryefficient short read aligner. Genome Biol 10:R25. 46.
68. Langmead B, Salzberg SL (2012) Fast gapped-read alignment with Bowtie 2. Nat Methods 9:357-359. 47.
69. Li H, Durbin R (2010) Fast and accurate long-read alignment with Burrows-Wheeler transform. Bioinformatics 26:589-595
70. Dirks RM, Pierce NA (2003) A partition function algorithm for nucleic acid secondary structure including pseudoknots. J Comput Chem 24:1664-1677.
71. Marçais G, Kingsford C (2011) A fast, lock-free approach for efficient parallel counting of occurrences of k-mers. Bioinformatics 27:764-770.
72. Quinlan AR, Hall IM (2010) BEDTools: A flexible suite of utilities for comparing genomic features. Bioinformatics 26:841-842

## Claims

1. A method of detecting a nucleic acid, comprising steps of
(a) providing a nucleic acid-containing compartment;
(b) hybridizing a plurality of single-stranded DNA oligonucleotide probes to nucleic acid molecules within said compartment;
(c) removing single-stranded DNA oligonucleotide probes from the compartment that have not specifically hybridized to any nucleic acid within the compartment;
(d) identifying single-stranded DNA-oligonucleotide probes specifically hybridized to nucleic acid molecules within said compartment by probe sequencing or probe amplification; and thus determining nucleic acids corresponding to the probe present in said compartment.
wherein the method does not comprise sequential probe hybridization as a means to amplify nucleic acid detection.

2. The method of claim 1, wherein the method does not comprise an RNA isolation step.

3. The method of any one of the preceding claims, wherein the method does not comprise a cDNA generation step.

4. The method of any of the preceding claims, wherein the nucleic acid is RNA, optionally, mRNA.

5. The method of any of claims 1-3, wherein the nucleic acid is ssDNA or dsDNA, optionally, ssDNA.

6. The method of any one of the preceding claims, wherein the nucleic acid-containing compartment is a eukaryotic cell, a nucleus of a eukaryotic cell, cytoplasm of a eukaryotic cell, a mitochondrion, a chloroplast, an exosome, a prokaryotic cell, a group of cells in a tissue, or a virus.

7. The method of any of the preceding claims, wherein the nucleic acid -containing compartment is sectioned before step (b).

8. The method of any of the preceding claims, wherein the nucleic acid -containing compartment or cell are biochemically separated or dissociated before step (b).

9. The method of any of the preceding claims, wherein step (b) is preceded by fixation of the nucleic acid-containing compartment, wherein, optionally, the nucleic acid-containing compartment is sectioned after fixation.

10. The method of any of claims 1 to 7, wherein the nucleic acid -containing compartment is not fixated, wherein it optionally is vitrified.

11. The method of any of the preceding claims, wherein the single-stranded DNA oligonucleotide probes comprise a target region complementary to a nucleotide sequence of a target nucleic acid flanked by a pair of universal primer regions,
wherein, optionally, the single-stranded DNA oligonucleotide probes further comprise a unique molecular identifier.

12. The method of any of the preceding claims, wherein the single-stranded DNA oligonucleotide probes specifically hybridize to a plurality of target nucleic acids present in the compartment.

13. The method of any of claims 1-4 and 6-11, wherein the single-stranded DNA oligonucleotide probes form a library that specifically hybridizes to substantially all mRNAs, optionally, substantially all RNAs present in the compartment.

14. The method of any of the preceding claims, wherein between step (c) and (d), an amplification of bound single-stranded DNA oligonucleotide probes is carried out.

15. The method of any of the preceding claims, wherein the bound single-stranded DNA oligonucleotide probes are identified by sequencing, preferably next generation sequencing.

16. The method of claim any of claims 1-13, wherein the bound single-stranded DNA oligonucleotide probes are identified by amplification, preferably by quantitative PCR.

17. The method of any of the preceding claims, further comprising spatially mapping detected nucleic acids in said compartment, comprising the steps of
(i) sectioning, cryosectioning, or cryomilling, preferably, cryosectioning, the compartment prior to step (b) to obtain a collection of fractions and thus separating nucleic acid molecules from each other depending on their localization;
(ii) identifying the single-stranded DNA-oligonucleotide probes specifically hybridized to nucleic acid molecules within each fraction in step (d) and thus determining the presence or absence of RNA corresponding to the probe in each fraction; and
(iii) mapping nucleic acids within the compartment.

18. The method of claim 17, wherein detection of nucleic acid, preferably RNA, is combined with the detection of at least one DNA locus within said compartment, comprising additional steps of
- determining the presence or absence of at least one DNA locus in each fraction, optionally, by sequencing, preferably by next generation sequencing; and
- determining co-segregation of said at least one DNA locus and the single-stranded DNA oligonucleotide probe(s) specifically hybridized to RNA.

19. Use of the method of any of the preceding claims for
(a) determining gene expression in single cells, groups of cells or intracellular compartments;
(b) identifying isoforms and allele-specific variants of RNAs within a compartment;
(c) quantifying transcription of genes; and
(d) identifying cell types of complex heterogenous tissue;
(e) identifying endogenous and exogenous dsDNA and ssDNA within a compartment
(f) mapping RNA location in the compartment
(g) mapping RNA and nucleic acid loci location in the compartment
(h) mapping RNA and protein location in the compartment
(i) mapping RNA, protein and nucleic acid loci location in the compartment.
